# EUROPEAN PATENT APPLICATION

(11) **EP 2 130 913 A1**
(43) Date of publication of application: **09.12.2009**
(21) Application number: 07715309.6
(22) Date of filing: 05.03.2007
(51) Int. Cl.: C12N 15/09, C12M 1/00, C12Q 1/68

(54) **CHIP FOR SAMPLING CELL COMPONENT, SYSTEM FOR ANALYZING CELL COMPONENT AND METHOD OF ANALYZING CELL COMPONENT USING THE SAME**

(71) Applicant: On-chip Cellomics Consortium, Tokyo 100-0006 (JP)
(72) Inventor: OKANO, Kazunori, Tokyo 160-0008 (JP); YASUDA, Kenji, Tokyo 135-0052 (JP)
(74) Representative: Prinz & Partner
(86) International application number: PCT/JP2007/054751
(87) International publication number: WO 2008/108004

(57) **Abstract**

The present invention provides a method, a chip device, and a system for quantitatively measuring intercellularly and intracellularly-localized mRNA and protein without loss of local space information. In the present invention, cell content is trapped atop a substrate or an electrode in the form of a two-dimensional projection of a cell. Accordingly, electrophoretic force is used, or the cellular moisture content is instantaneously evaporated and immobilized. mRNA immobilized to a two-dimensional surface is identified with a labeled probe which hybridizes to the mRNA.

## Description

### TECHNICAL FIELD

The present invention relates to a cell component analysis chip, a cell component analysis system, or method therefor for the quantitative measurement of mRNA or protein which is a gene product expressed at a cell or a tissue that is a cell aggregate in addition to space localization information of mRNA or protein at cells or tissues that are cell aggregates.

### BACKGROUND ART

As progress in the genome project is made, efforts to understand living organisms at the DNA level, diagnose diseases, and comprehend life phenomena are intensifying. Investigating gene expression is an effective way to comprehend life phenomena and investigate the workings of genes. This useful method uses a DNA probe array in which a multitude of DNA probes are divided by type and immobilized atop a solid surface, or, alternatively, a DNA probe chip (also called an oligo chip because oligonucleotide derivatives are what is actually immobilized). Protein chip concepts in which antibodies and antigens (instead of DNA probes) are immobilized atop substrates in an array are also in practical use. In one example of an allergen detection system in actual medical testing use, allergens are detected by reacting a serum specimen with a device upon which tens or hundreds of antigens (allergens) that will cause an allergy are immobilized on a substrate, specifically trapping the IgM in the serum that causes an antigen-antibody reaction with each allergen, reacting enzyme-labeled secondary antibodies that bond with IgM, and measuring enzyme activity by combining active enzymes with chemiluminescence.

DNA chip fabrication methods include that in which lithography, which is often used in photochemical reactions and the semiconductor industry, is used to synthesize the designed oligomer sequences into the multiple partitioned cells one base at a time (Science 251, 767-773 (1991)) or, alternatively, a method in which DNA probes and protein probes are implanted in each cell one at a time (Proc. Natl. Acad. Sci. USA 93, 4613-4918 (1996)).

These chips all have a structure in which multiple probes are arranged in an array on the surface of a glass slide, etc. that is divided into sections. Which probe is at which position is generally indexed only by the physical position at which the probe is immobilized.

In the vast majority of detection cases, fluorochrome is used as a labeling substance for DNA chips or the like. With protein chips, although there are many cases in which enzyme labeling substances are used and detection is performed with chemiluminescent or bioluminescent systems as described above, diverse methods such as fluorescent labeling detection and electrochemical luminescent detection are also in active use.

In the electrochemical method, antigen trapping antibodies exist at the electrode surface. Ruthenium complex is used as a labeling substance for sandwich-use antibodies (Clin. Chem., 37, 1534-1539 (1991)). The ruthenium electrons that entered an excited state when ruthenium on the electrode surface oxidized and then reduced when coupled with the TPA redox reaction return to the ground state and emit light.

### DISCLOSURE OF THE INVENTION

With the above-mentioned conventional art, methods for the identification and detection of mRNA and proteins, which are the respective products of gene transcription and translation performed using a DNA probe chip or a protein chip, were thoroughly investigated, and measurement can be performed if a sample in the form of a solution can be prepared through some kind of method. Serum samples are used in the allergen testing from serum specimens explained in the above-mentioned conventional art. In mRNA testing, the RNA component is extracted in advance from an adequately large cell specimen, reverse transcriptase is used to synthesize cDNA by means of a reverse transcription reaction, and amplification is performed by means of PCR amplification and cRNA synthesis. Further, labeling dNTP is introduced and a large quantity of labeling DNA chains are obtained during complimentary chain synthesis. Recently, the mainstream method is one in which aminoallyl dNTP is introduced and then fluorochrome is reacted with the amino group to obtain fluorescently-labeled DNA chains. The thusly obtained fluorescently-labeled DNA chains are, in a manner of speaking, hybridized with the DNA chip and analysis of various types of expressed DNA is performed. Thus, analysis is only possible if a solution sample is obtained in advance.

When handling cells, which constitute a large percentage of specimens, there are instances in which it is problematic to convert the cell component being measured to a solution state. Since balance of the whole is achieved through mutual interaction between a cell and adjacent cell groups in multi-cellular organisms, pulverizing cell groups will result in a uniform state, causing localized information for each cell to be lost. Further, even within individual cells, mRNA, which is an expressed gene, is localized. Proteins, which are the final product, are also localized within the cell in accordance with their function. These local mRNA and proteins change in accordance with a cell's state of activity, in other words the level of communication between a cell and the adjacent cell group, and the environment in which the cell is placed. Therefore, if an attempt is made to obtain information useful for medically-directed testing drug discovery, and to more accurately describe biological functions, the mRNA and proteins localized between or inside cells must be quantitatively detected in a state in which local space information is retained.

In other words, the purpose of the present invention is to provide a cell component analysis chip, cell component analysis system, and method that makes it possible to overcome the above-mentioned problems, and quantitatively measure the mRNA and proteins localized between or inside cells without losing local space data.

Accordingly, the present invention provides the following cell component analysis chip, cell component analysis system, cell component analysis device, and cell component analysis method:
(1) A cell component analysis system comprising:
   a substrate structure wherein probes for the capture of a predetermined biological substance are anchored to a substrate surface;
   a spatial structure that retains a cell-containing buffer on a surface upon which the probes for the capture of a biological substance of the substrate structure are provided;
   a mechanism that destroys cells in the buffer retained in the spatial structure;
   a mechanism that enables two-dimensional projection of the predetermined biological substance contained in the destroyed cell onto the substrate surface; and
   a mechanism that enables distinction between and detection of biological substances trapped by a probe on the substrate surface.
(2) A cell component sorting chip for cell component analysis comprising:
   a substrate having a region divided into predetermined sections;
   a means that enables communication between the regions of the substrate as necessary;
   probes provided on the surface of the region of the substrate, said probes being able to trap the components of a cell contained in said region; and
   electrodes that apply an electrical field from above and below the regions of the substrate.
(3) The cell component sorting chip according to (2) wherein the surface of the divided regions is a polymer layer prepared by the polymerization of two types of monomer, the polymer layer is a structure polymerized such that the first monomer having the first polymerization functional group in two locations and the second monomer having the second polymerization functional group in two locations are arranged alternately, and either the first monomer or the second monomer has a biological substance trapping residue.
(4) The cell component sorting chip according to (2) wherein the surface of the divided regions is a polymer layer prepared by the polymerization of two types of monomer, the polymer layer is a structure polymerized such that the first monomer having the first polymerization functional group in two locations and the second monomer having the second polymerization functional group in two locations are arranged alternately, and both the first monomer and the second monomer have a biological substance trapping residue.
(5) The cell composition substance sorting chip according to (3) and (4) wherein the polymer layer is formed on the electroconductive member surface provided on the substrate surface.
(6) The biological substance analysis system according to (1) wherein the mechanism that two-dimensionally projects the destroyed cell containing the predetermined biological substance comprises a power source and electrodes for application of an electrical field on either side of a spatial structure for retention of a buffer containing the cell.
(7) The biological substance analysis system according to (1) wherein the mechanism that destroys the cells is capable of ultraviolet irradiation of the cells at a wavelength of 320 nm to 400 nm.
(8) The biological substance analysis system according to (1) wherein the mechanism that enables distinction between and detection of biological substances trapped in the probes on the substrate surface is nanoparticles that label the labeled probe that will hybridize to the biological substance and a measurement means for identifying the nanoparticles wherein the nanoparticles are specified by size and material.
(9) The biological substance analysis system according to (1) wherein the surface of the substrate to which the biological substance trapping probe is immobilized is prepared by the polymerization of two types of monomer, the polymer layer is a structure polymerized such that the first monomer having the first polymerization functional group in two locations and the second monomer having the second polymerization functional group in two locations are arranged alternately, and either the first monomer or the second monomer has a biological substance trapping residue.
(10) A cell component sorting method wherein a cell component sorting chip for cell component analysis provided with a substrate having a region divided into predetermined sections, a means for communicating between the regions of the substrate as necessary, probes provided on the surface of the region of the substrate that can trap the components of a cell contained in said region, and electrodes that apply an electrical field from above and below the regions of the substrate is used, said method comprising the following steps:
   retaining a cell-containing buffer solution in a region of the section;
   destroying the cells in the region of the section;
   two-dimensionally projecting the predetermined biological substance contained in the destroyed cells onto a region of the substrate surface and trapping a predetermined substance in the probes;
   reacting a probe labeled with predetermined nanoparticles with the captured predetermined substance; and
   analyzing the particle diameter and elemental composition of the nanoparticles of the reacted probe to identify the type and position of each molecule of the predetermined biological substance.
(11) A cell component sorting chip comprising a substrate having a region divided into predetermined sections for retaining individual cells, multiple types of probes capable of capturing the cell components arranged in spots on the divided region of the substrate, and a mechanism that enables communication between the regions of the substrate as necessary.
(12) A cell component analysis method wherein a cell component sorting chip provided with a substrate having a region divided into predetermined sections for retaining individual cells, multiple types of probes capable of capturing the cell components arranged in spots on the divided region of the substrate and a mechanism that enables communication between the regions of the substrate as necessary, and a cell component analysis chip for cell component analysis provided with electrodes for the application of an electrical field from above and below the regions of the substrate are used, said method comprising the following steps:
   retaining a cell-containing buffer solution in a region of the section;
   destroying the cells in the region of the section;
   capturing the predetermined substance contained in the destroyed cells with the plurality of probes that exist in the regions of the substrate;
   reacting a probe labeled with predetermined nanoparticles with the captured predetermined substance; and
   analyzing the particle diameter and elemental composition of the nanoparticles of the reacted probe to identify the type and position of each molecule of the predetermined biological substance.
(13) A cell component analysis method wherein a cell component sorting chip for cell component analysis provided with a substrate having a region divided into predetermined sections and a means for communicating between the regions of the substrate as necessary is used, said method comprising the following steps:
   retaining a cell-containing buffer solution in a region of the section;
   drying the cells retained in the section of the region by exposing said cells to a dry atmosphere of predetermined requirements or freezing and then drying the section of the region to which the cells implanted,
   adding a predetermined solution to make the cell membrane permeable,
   washing substances other than cell components immobilized to the section of the region;
   reacting probes labeled with predetermined nanoparticles with cell components immobilized to the section of the region; and
   analyzing the particle diameter and elemental composition of the nanoparticles of the reacted probe to identify the type and position of each molecule of the predetermined biological substance.
(14) A cell component analysis method wherein a cell component sorting chip for cell component analysis provided with a substrate having a region divided into predetermined sections and a means for communicating between the regions of the substrate as necessary is used, said method comprising the following steps:
   retaining a cell-containing buffer solution in a region of the section;
   exposing cells trapped in the section of the region to a solution containing aldehydes to immobilize the cell components;
   adding a predetermined solution to make the cell membrane permeable,
   washing cell components immobilized to the section of the region;
   reacting probes labeled with predetermined nanoparticles with cell components immobilized to the section of the region; and
   analyzing the particle diameter and elemental composition of the nanoparticles of the reacted probe to identify the type and position of each molecule of the predetermined biological substance.
(15) A cell component analysis method wherein a cell component sorting chip for cell component analysis provided with a substrate having a region divided into predetermined sections to which predetermined markers are immobilized, a means for communicating between the regions of the substrate as necessary, probes provided on the surface of the region of the substrate, said probes being able to trap the components of a cell contained in said region, and electrodes for the application of an electrical field from above and below the regions of the substrate is used, said method comprising the following steps:
   retaining a cell-containing buffer solution in a region of the section;
   obtaining a first image wherein the cells retained in the region of the section and the marker fit into a single screen;
   destroying the cells in the region of the section;
   two-dimensionally projecting the predetermined biological substance contained in the destroyed cells onto a region of the substrate surface and trapping a predetermined substance in the probes;
   reacting probes labeled with predetermined nanoparticles with the captured predetermined substance;
   obtaining a second image wherein the probe-labeling nanoparticles and the marker fit into a single screen, and
   analyzing the particle diameter and elemental composition of the nanoparticles of the reacted probe to identify the type and position of each molecule of the predetermined biological substance; and
   matching the markers of the first image and the second image, and superimposing said images.
(16) A cell component analysis method wherein a cell component sorting chip for cell component analysis having a substrate having a region divided into predetermined sections to which predetermined markers are immobilized, said substrate provided with a means for communicating between the regions of the substrate as necessary is used, said method comprising the following steps:
   retaining a cell-containing buffer solution in a region of the section;
   obtaining a first image wherein the cells retained in the region of the section and the marker fit into a single screen;
   drying the cells retained in the section of the region by exposing said cells to a dry atmosphere of predetermined requirements or freezing and then drying the section of the region to which the cells implanted;
   adding a solution containing pepsin to make the cell membrane permeable;
   washing cell components immobilized to the section of the region;
   reacting probes labeled with predetermined nanoparticles with cell components immobilized to the section of the region;
   obtaining a second image wherein the probe-labeling nanoparticles and the marker fit into a single screen,
   analyzing the particle diameter and elemental composition of the nanoparticles of the reacted probe to identify the type and position of each molecule of the predetermined biological substance; and
   matching the markers of the first image and the second image, and superimposing said images.
(17) A cell component analysis system comprising:
   a substrate structure wherein a probe for the capture of a predetermined biological substance is immobilized to a substrate surface;
   a mechanism coated with an agarose thin film for prevention of cell adsorption to the substrate surface upon which the probes are immobilized;
   a spatial structure that retains a cell-containing buffer on a surface upon which the probes for the capture of a biological substance of the substrate structure are provided;
   a mechanism that destroys cells in the buffer retained in the spatial structure,
   a mechanism that enables two-dimensional projection of the predetermined biological substance contained in the destroyed cell onto the substrate surface, and
   a mechanism that enables distinguishing and detecting biological substances trapped by a probe on the substrate surface.
(18) A cell component sorting chip comprising:
   a substrate having one or more sections that contain biological substance trapping probes, wherein the section is defined by (a) the first surface on the substrate that has affinity or non-affinity to the cell, (b) the side walls that have affinity to the cell and are arranged on the first surface, and (c) the second wall facing the first surface via a spacer, and said section has a volume capable of retaining sample solution containing at least a single cell;
   the biological substance trapping probes are immobilized to the first surface, the side walls are as high or lower than the spacer, and
   a pair of electrodes for the application of an electric field is provided the first surface and the second surface.
(19) The cell component sorting chip according to (18) wherein each of the multiple sections is connected to the neighboring section so as to allow fluid communication.
(20) The cell component sorting chip according to (18) wherein the pair of electrodes are parallel plate electrodes, the first surface is formed from one surface of one of the electrodes, and the second surface is formed from one surface of the other electrode.
(21) The cell component sorting chip according to (18) wherein the first surface that has affinity to the cell comprises a polymer layer containing the biological substance trapping probes.
(22) The cell component sorting chip according to (18) wherein the first surface that has non-affinity to the cell comprises a polymer layer containing the biological substance trapping probes and a layer of agarose gel applied on the polymer layer.
(23) The cell component sorting chip according to (22) wherein the agarose concentration does not exceed 0.05%.
(24) The cell component sorting chip according to (21) or (22) wherein the polymer layer contains a heteropolymer comprising two types of monomer alternately bonded and the biological substance trapping probes are bonded to one of the two types of monomer.
(25) The cell component sorting chip according to (18) wherein a plurality of spots to which biological substance trapping probes are immobilized are formed on the first surface of the interior of each section on the substrate.
(26) The cell component sorting chip according to (18) wherein the side walls that have non-affinity to the cell are formed from agarose gel or light curing resin.
(27) The cell component sorting chip according to (18) wherein the pair of electrodes are for the application of an electrical field to move the biological substance being tested towards the biological substance trapping probes.
(28) The cell component sorting chip according to one of (18) to (27) wherein the plurality of types of biological substance probes are immobilized to the first surface in accordance with the biological substance being tested.
(29) A device for cell component analysis that uses a biological substance sorting chip according to one of (18) to (28) comprising:
   a means for placement of the biological component retaining the test solution containing at least one cell at the interior of the section;
   a means for irradiating the interior of the section with UV light having a wavelength absorbed by the cell in order to destroy the cell at the interior of the section and force emission of the cell content that contains the biological substance being tested;
   a power source that supplies electric power between the pair of electrodes; and
   a means for detecting and/or quantifying a biological substance trapped at the first surface.
(30) The device according to (29) wherein the detection and/or quantitative means contains an imaging device, electron scanning microscope, or atomic force microscope provided with a CCD camera.
(31) A system for the analysis of cell components comprising:
   the cell composition sorting chip according to one of (18) to (28);
   a means for irradiating the interior of the section with UV light having a wavelength absorbed by the cell in order to destroy the cell at the interior of the section and force emission of the cell content that contains the biological substance being tested;
   a power source that supplies electric power between the pair of electrodes; and
   a means for detecting and/or quantifying a biological substance trapped at the first surface.
(32) A method of cell component analysis using a biological component sorting chip according to any one of (18) to (28) comprising the following steps:
   (i) injecting sample solution containing at least a single cell into the section;
   (ii) irradiating the interior of the section with UV light having a wavelength absorbed by the cell in order to destroy the cell at the interior of the section and force emission of the cell content that contains the biological substance being tested;
   (iii) using the net electric charge of the biological substance being tested to apply an electrical field between the first and second surfaces, moving the biological substance emitted from the cell towards the biological substance probes, and trapping the biological substance with the probes;
   (iv) washing away cell content other than the biological substance trapped in the probes;
   (v) using a labeling substance to label the biological substance trapped in the probes, and
   (vi) detecting and/or quantifying the labeled biological substance under a microscope.
(33) The cell component analysis method according to (32), wherein the plurality of biological substance trapping probes use the cell component sorting chip immobilized to the first surface, the multiple types of biological substance trapped in probes in steps (v) and (vi) are labeled with a different labeling substance according to type, and the different labeled substances are detected and/or quantified and the biological substance being tested is thus identified.
(34) A cell component analysis method according to (32) wherein a cell component sorting chip having a first surface that has affinity to the cell is used, said method comprising the following steps:
   prior to step (ii), recording image (a), which shows the image projected substantially vertically onto the first surface of the cell that is adhered to the first surface that has affinity to the cell;
   after step (v), recording image (b), which shows the same range as image (a); and,
   in step (vi), obtaining position information regarding various biological substances inside the cells by superimposing image (a) and image (b).
(35) A cell component analysis method wherein a cell component sorting chip having a substrate provided with 1 or more sections for the retention of sample solution is used, the section is defined by (a) the first surface which is atop the substrate and has affinity with the cell and (b) the side walls which are arranged atop the first surface and do not have affinity with the cell, and has a volume capable of retaining sample solution containing at least a single cell,
   said cell component analysis method comprising the following steps:
   (i) injecting sample solution containing at least a single cell into the section;
   (ii) dry the sample solution in the section, thereby adhering the cell to the first surface,
   (iii) treating the cell with an enzyme to make the cell membrane permeable to the labeled probes;
   (iv) using labeled probes that specifically bond with the biological substance being tested to label the biological substance in the cell; and
   (v) detecting and/or quantifying the labeled biological substance.
(36) The method according to (35) wherein the first surface contains a polymer layer having a positive charge.
(37) A method according to (35) wherein step (ii) comprises exposing the sample solution to a dry helium atmosphere or freeze drying the sample solution with liquid nitrogen.
(38) The cell component analysis method according to (35) comprising the following steps:
   following step (ii), recording image (a), which shows the image projected substantially vertically onto the first surface of the adhered cell;
   after step (iv), recording image (b), which shows the same range as image (a); and,
   in step (v), obtaining position information regarding various biological substances inside the cells by superimposing image (a) and image (b).
(39) A method according to (35) to (38) wherein the sample solution contains cells or histological sections.

### BRIEF DESCRIPTION OF THE DRAWINGS

Fig. 1(A) is a plan view showing the cell component sorting chip of Example 1. Fig. 1 (B) is a cross-section showing the view from the direction of the arrows in the A-A position of Fig. 1(A). (C) is another example of a cross-section showing the view from the direction of the arrows in the A-A position of (A).
Fig. 2 relates to a cell component sorting ship mainly for use with floating cells. Fig. 2(A) shows a cross-section of a cell inside a section 5 sandwiched between two substrates (substrates 1 and 1'). Fig. 2(B) is a diagram schematically showing the process in which pulse laser 21 destroys the cells within the section 5s and an electrical charge (1.2 V) is simultaneously applied by power source 60 between electrodes 2 and 2', pulling nucleic acid 23 such as mRNA, DNA, etc. towards electrode 2. Fig. 2(C) is a diagram schematically showing the situation in Fig. 2(B) after the passage of time.

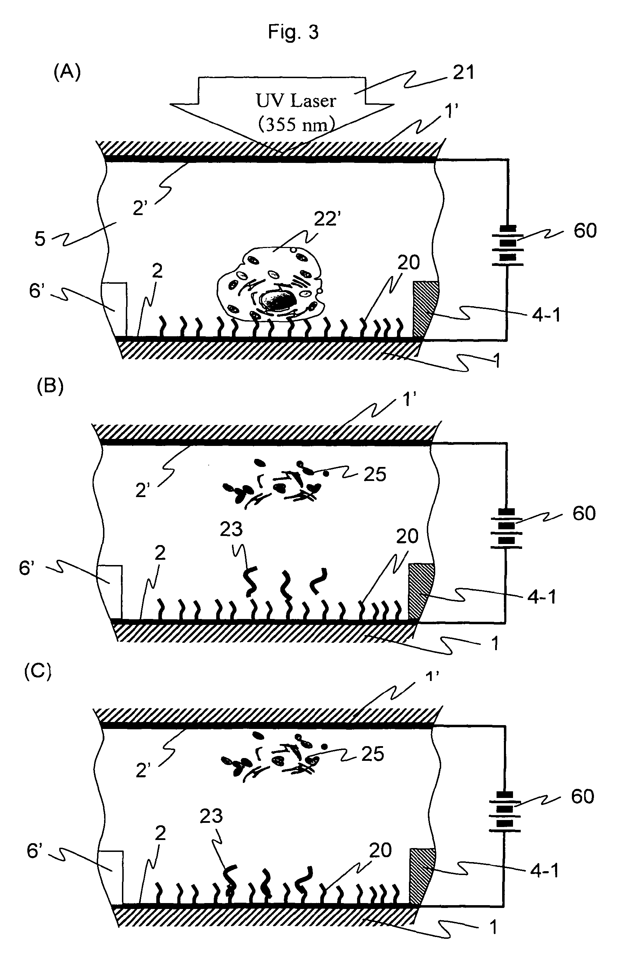

Fig 3 relates to a cell component sorting chip for cells with attachment properties. Fig. 3(A) is a cross-section diagram showing a cell inside a section 5 sandwiched between two substrates (substrates 1 and 1'). Fig. 3(B) is a diagram schematically showing the process in which pulse laser 21 destroys the cells within the section 5s and an electrical charge (1.2 V) is simultaneously applied by power source 60 between electrodes 2 and 2', pulling nucleic acid 23 such as mRNA, DNA, etc. towards electrodes 2 and peeling proteins and the like from the surface of the substrate. Fig. 3(C) is a diagram schematically showing the situation in Fig. 3(B) after time has passed and nucleic acid 23 of mRNA, DNA, etc. has hybridized to polyT probe 20 on the substrate.
Fig. 4 explains the method for identifying the mRNA groups trapped in polyT probe 20 on the surface of electrode 2.
Fig. 5 is a diagram schematically showing that the relationship between labeling substances 31', 32' and 33' of the mRNA anchored to probe 20 on electrode 2 and the outline 41 of the cell 22 can be recognized on a two-dimensional surface.
Fig. 6 is a diagram systematically showing another example of a mRNA labeling substance anchored on electrode 2.
Fig. 7(A) is a plan view showing the cell component sorting chip of Example 2, while Fig. 7(B) is a cross-section showing the view from the direction of the arrows in the A-A position of Fig. 7(A).
Fig. 8 is a diagram systematically showing another example of a mRNA labeling substance anchored on a substrate.
Fig. 9(A) is a plan view showing the cell component sorting chip of Example 3, while Fig. 9(B) is a cross-section showing the view from the direction of the arrows in the A-A position of Fig. 9(A).
Fig. 10 is a plan view showing the cell component sorting chip of Example 3.
Fig. 11 is a plan view showing an example of a variant of the cell component sorting chip of Example 3.

### BEST MODE FOR CARRYING OUT THE INVENTION

According to one embodiment of the present invention, a cell component sorting chip containing a substrate with one or more sections containing cell substance trapping probes, a kit, a system, and a method that use said chip to analyze cell components are provided. In the above-mentioned chip of the present invention, the above-mentioned section is defined by a first surface on the above-mentioned substrate having affinity or non-affinity to the cell, a side wall having non-affinity to the cell arranged on the above-mentioned first surface, and the above-mentioned first surface and an opposing second surface separated by a spacer, and has a volume enabling the retention of a sample solution containing at least one cell. The above-mentioned biological substance trapping probe is immobilized to the above-mentioned first surface, and the above-mentioned side wall is the same height or lower than the above-mentioned spacer. In an embodiment of the present invention, the cell component sorting chip is provided with a pair of electrodes for the application of an electrical field between the above-mentioned first surface and the above-mentioned second surface.

Throughout the present specification, "biological substance" means substances to be tested in biological samples such as polynucleotides such as DNA and RNA (for example, mRNA, which is a gene transcription product), proteins or peptides, which are DNA translation products, or antibodies, antigens and the like.

Throughout the present specification, "biological substance trapping probes" function as a bonding means for trapping a biological substance on the above-mentioned first surface and may be a polynucleotide, polypeptide, antibody, antigen, etc., depending on biological substance type. For example, if the biological substance is mRNA, a polynucleotide having a complimentary sequence that can hybridize thereto can be used as the biological substance trapping probe. Alternatively, if the biological substance is an antigen, an antibody that specifically binds thereto can be used as a biological substance trapping probe. The biological substance capture probe can typically be immobilized to the above-mentioned first surface, for example by bonding through an anchoring means such as a specific functional group.

The plurality of sections on the cell component sorting chip of the present invention are, when necessary, connected to allow fluid communication between the adjacent section.

In the cell component sorting chip of the present invention, the first surface that has affinity to the cell is preferably formed from a polymer layer containing the biological substance trapping probe. The first surface that has non-affinity to the cell, can be formed by, for example, application of an agarose gel layer on the polymer layer containing the biological substance trapping probe.

Throughout the present specification, a surface that has "affinity" to a cell means a surface having a property whereby a cell substantially adsorbs thereon; a surface that has "non-affinity" to a cell means a surface having a property whereby cells do not substantially adsorb thereon. A cell "substantially adsorbing" or "not substantially adsorbing" to a surface means that when the chip of the present invention is used and a cell is handled in accordance with the usual routine, that a cell will adsorb to a surface with affinity to the cell but will not adsorb to a surface that does not have affinity to the cell to the extent that one skilled in the art will be able to discern "adsorption" or "non-adsorption" to that surface.

Throughout the present specification, the term "polymer" means a chemical polymer from which the polymer layer is composed. An example of a typical polymer used in the present invention is a heteropolymer formed from polymerization of the smallest repeating unit of a dimer formed from a first monomer and a second monomer. In one embodiment, the biological substance trapping probe used in the present invention is bonded to either the first or second monomer of this sort of heteropolymer through a specific functional group. Throughout the present specification, the term "polymer layer containing a biological substance trapping probe" typically means a polymer layer formed from a polymer bonded to the biological substance trapping probe.

In one embodiment of the present invention, the "pair of electrodes" is used to move the biological substance being tested towards the above-mentioned biological substance trapping probes so that the above-mentioned electrical field can be applied. In other words, in one example of the method of the present invention in which the cell component sorting chip of the present invention is used to analyze cell components, (i) sample solution containing at least one cell is injected into the above-mentioned section; (ii) in order to destroy the cell inside the above-mentioned section and force the emission of the intracellular content containing the biological substance being tested, the interior of the above-mentioned section is irradiated with UV light having a wavelength absorbed by the cell; (iii) the net electrical charge of the biological substance being tested is used to apply an electrical field between the above-mentioned surfaces 1 and 2, forcing the above-mentioned biological substance emitted from the above-mentioned cell to migrate towards the above-mentioned biological substance trapping probe and be trapped thereon; (iv) intracellular biological substances other than the above-mentioned biological substance trapped in the above-mentioned probe are washed away; (v) the above-mentioned biological substance trapped in the above-mentioned probe is labeled; and (vi) the above-mentioned labeled biological substance is detected and/or quantified under a microscope.

In a preferred embodiment, a cell component sorting chip, upon whose first surface is immobilized multiple types of biological substance capture probe, is used such that multiple biological substances trapped by the above-mentioned biological substance trapping probes are labeled with different labeling substances depending on type, detected and/or quantified based on the different labeling substances in the above-mentioned steps (v) and (vi), and identified.

Further, before the above-mentioned step (ii), image (a), which shows the cell that is immobilized to the above-mentioned first surface that has affinity thereto, is projected substantially perpendicularly to the above-mentioned first surface, is recorded. After the above-mentioned step (v), image (b), which covers the same range as the above-mentioned image (a), is recorded. In the above-mentioned step (vi), image (a) and image (b) are superimposed, making it possible to obtain positional information on biological substances inside the cells.

In the present invention, first, in order to minimize decomposition of mRNA or expressed proteins and realize a method in which it is not necessary to dilute concentration, cell content is trapped on a substrate or on an electrode with the cell in the form of a two-dimensional projection.

For this to be achieved, electrophoretic force can be used. Thusly, a cell is held at the extremely narrow space between two electrodes, and by applying an electrical field, the cell is destroyed as the content of the cell is simultaneously pulled toward the electrode surface. Alternatively, using UV light to destroy cells is also effective. UV light of approximately 350 nm is not absorbed by the solvent (water), and is absorbed by the cell. Therefore, the cell can be made soluble without boiling the solvent. In this instance as well, electrophoretic force is used to trap the biological substance content in a two-dimensionally projected form.

In any cell destruction method, it is crucial that the electric line of force be formed in a substantially perpendicular direction to the electrode surface to trap the content of biological substances in the form of a two-dimensional projection. Thusly, the electrode surface area must be adequately wide in comparison with the size of the cell. In addition to these conditions, if the distance between electrodes is approximately several dozen µm, thereby making it difficult for convective flow to occur, the biological substances inside the cell will migrate in an almost perpendicular direction to the electrode surface. On the substrate or electrode surface upon which the biological substance content will be trapped, a substance for bonding with mRNA, such as polyT, is immobilized in advance for the purpose of trapping mRNA. For example, for proteins, a surface to which cations such as amino groups or anions such as carboxyl groups are densely introduced is used to electrostatically immobilize proteins. Alternatively, the electrode surface is covered with hydrophobic residue and using affinity of hydrophobic sections of proteins that associate by the expulsion energy of water molecules, the immobilization of all the proteins to the substrate surface is enabled.

If the biological substance to be measured can be predicted, more accurate measurement can be enabled by immobilizing corresponding DNA probes to the substrate in advance. For example, the intracellular distribution of mRNA derived from homo sapiens paraoxonase 1 (PON 1) can be measured if the sequence of SEQ ID NO: 1 immobilized as a probe is used. Further, by immobilizing a plurality of DNA probes on the substrate, a plurality of mRNA sequences to be measured can be trapped on the substrate.

CGAAGCACCT CTGATGCAGG AGGATTCTCT GAGTCATAGA
AGAAGATTTT : (SEQ ID NO: 1)

The present invention can also be applied to specimens which are tissue-like aggregates of cells. However, tissue-like specimens with a single cell layer are preferable. For example, 10 cells lined up in a row shall be considered a hypothetical tissue-like specimen. If an electrical field is applied while the specimen is in between the two electrodes and being bombarded with UV, individual cell-derived biological substances will, in accordance with individual cells, immobilize to the electrode surface while retaining two-dimensional information.

As the second method for trapping and immobilizing biological substance content on a substrate, it is effective to instantly evaporate the water content of cells without use of electrodes. When this method is used, mRNA and proteins can be immobilized without disturbing cellular position because the cell is exposed to high temperature and its water is evaporated while it is located on the substrate. Intracellular mRNA and proteins can, of course, be immobilized while retaining two-dimensional information. Alternatively, intracellular mRNA and proteins can be instantly frozen with liquid nitrogen and then dried. Cells can also be immobilized through the use of reagents such as formaldehyde and glutaraldehyde. When formaldehyde and glutaraldehyde are used to perform immobilizing, analysis of mRNA in particular is achieved by the following procedure: cells are immobilized through the use of formaldehyde; a protein degradative enzyme such as trypsin is used to destroy the cell membrane surface and structural proteins such that the intracellular mRNA can hybridize to mRNA to which the nanoparticle labeling probes that will be explained later are immobilized; and the nanoparticles that reacted are analyzed at a two-dimensional surface level. All of the methods make the subsequent detection of mRNA and proteins possible.

In the second immobilization method, mRNA and proteins are immobilized on the substrate simultaneously. Since cell destruction and immobilization take place within a short period of time, it is not necessary to pay too much attention to the mRNA degradation caused by RNase that can present a problem during mRNA detection. After immobilization, the substrate is washed with an RNase inhibitor, and more accurate mRNA detection becomes possible if operations are performed to remove RNase activity. Typical proteins are often immobilized in a denatured state, so it is not necessary to pay too much attention to degradation caused by protease.

In the second immobilization method, genomes are simultaneously immobilized on the substrate in addition to mRNA and proteins. Consequently, by reacting the forgoing with probes having nanoparticles that correspond thereto, quantitative data having spatial information on mRNA and protein expression of the same cells on the same substrate can be obtained. Specific genes of the genome that exists in the nucleus can also be distinguished from the mRNA and simultaneously detected.

In the present invention, no matter which immobilization method is used, intracellular substances are immobilized on a two-dimensional surface, in this case a substrate or electrode, while retaining position information. In this case, if the intracellular substances in a cell are two-dimensionally immobilized, biological substances cannot be identified as they are with a DNA chip or protein chip, wherein a different probe is immobilized to each section element and the biological substance being searched for is identified on the basis of the position of the element. Therefore, labeled probes that selectively bond to intracellular substances bonded to two-dimensional surfaces are used, appropriate identifiers are provided, and the bonded cell content is thereby identified.

When two-dimensionally immobilizing intracellular substances, if the purpose is obtaining position information sufficient to enable measurement of differences between individual cells, individual cells are retained in individual sections while maintaining cell interaction, and, two-dimensional position information and quantity can be measured at the cellular level of each biological substance by arranging multiple probes to the spots in each section as with a conventional DNA microarray or protein chip. In this instance, as in instances in which the intracellular substances are two-dimensionally projected and measured, cell position is maintained, and labeling probes are provided with appropriate identifiers that selectively bond to intracellular substances that are, in turn, bonded with affinity to a plurality of probes arranged in cell immobilization sections, and are used to identify the immobilized intracellular substances.

Appropriate identifiers are, for example, fluorescent molecules, which are detected on a single molecule basis. It is even easier to use fluorescent nanoparticles as an identifier and counting the fluorescent points on a substrate. Differentiation of the targets of measurement can be achieved by differentiating the fluorescent bodies used to label the corresponding probes on the basis of type and strength. Alternatively, nanoparticles are used. Nanoparticles whose particle diameter and elemental composition vary with the target of measurement are used. Therefore, intracell distribution can be quantitatively measured while distinguishing between the target of measurement by counting the type and quantity of nanoparticles added to the probes that reacted to the surface on which the cell is two-dimensionally projected.

Due to the present invention, position information and quantity of intracellularly-expressed mRNA and proteins in addition to genomes bearing their information within cells and between cells can be detected at the molecular level.

The average animal cell size is approximately 10 to 30 µm. Within these animal cells exist in excess of 20 thousand genes, with at least dozens of molecules and several hundred mRNA expressed. There are a few types of animal cell that consist of over 100,000 molecules. Many genes expressed in large quantities are housekeeping genes, of which an almost regular quantity are constantly expressed within cells. Accordingly, the genes targeted by the present invention are fairly small in number and have a number of expressed mRNA molecules that fluctuates when placed in a cell.

If, for example, 100 molecules are expressed, with a method such as a conventional DNA chip, detection is not possible without amplification. This is because during the step in which cells are pulverized and mRNA is extracted, degradation occurs, concentration thins, and hybridization requires an amount of time that is, kinetically speaking, nearly infinite.

In the present invention, the content of a cell is directly trapped on a substrate or on an electrode in the form of a 2-D projection. Hereinbelow, examples are explained in detail with drawings as a reference.

### EXAMPLES

### [EXAMPLE 1]

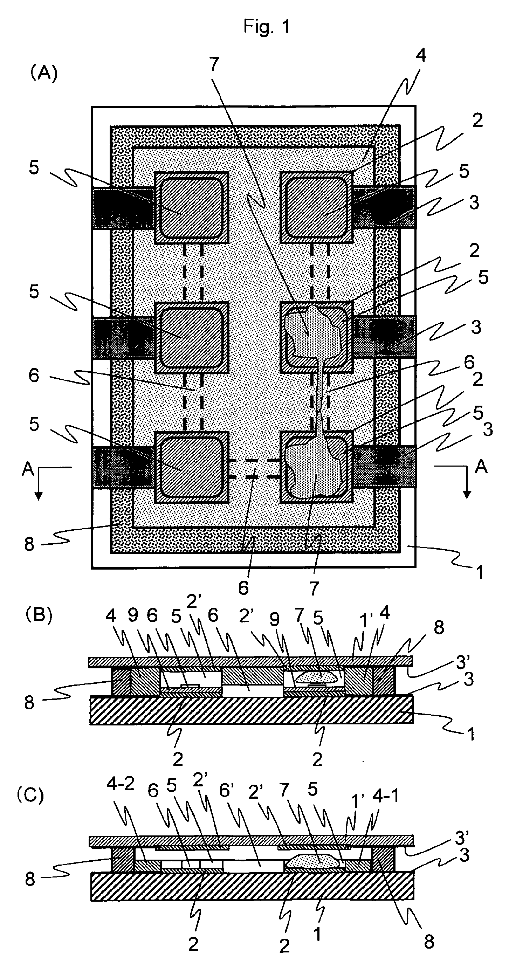

Fig. 1(A) is a plan view showing the cell component sorting chip of Example 1. Fig. 1 (B) is a cross-section showing the view from the direction of the arrows in the A-A position of Fig. 1(A). (C) is another example of a cross-section showing the view from the direction of the arrows in the A-A position of (A). 1 is a quartz substrate upon which agarose gel film 4 with a thickness of 5 µm is formed, and sections 5 for cell retention are prepared at a predetermined interval. The size of the sections 5 is, for example, 30 µm square. When the average size of the above-mentioned animal cell is 10 to 30 µmϕ, the size of the sections 5 may be 9 times this size or the equivalent size. Fig. 1 shows cells 7 arranged in multiple sections 5. If necessary, each section 5 is connected by a channel 6, making it possible for cells 7 that are retained in the sections 5 to interact. Transparent electrodes 2 formed from indium tin oxide (IDO) are located on the top surface of substrate 1 in a position that corresponds to each section 5. Transparent electrodes 2 may be prepared by vapor depositing gold, platinum, or chrome until transmitted light of approximately 30 to 70% is achieved. Electrodes 2 are regarded as approximately the same size as sections 5. Glass substrate 1' is provided on the upper surface of the abovementioned agarose gel membrane 4. The structure of substrate 1' is such that a regular interval is maintained by spacer 8. Spacer 8 is installed on substrate 1' in advance and is mounted on glass substrate 1 by pressing. In this case although it is preferable to remove agarose gel in advance from the portion of substrate 1 that will contact the spacer, if agarose gel 4 is thin as in (C), the spacer may also be pressed into place from above the gel. Opposing electrodes 2' are provided in the section 5 position on glass substrate 1'. Extraction electrodes 3, which are connected to electrodes 2 and opposing electrodes 2', are provided on quartz substrate 1 and glass substrate 1'. Since agarose gel film 4 of quartz substrate 1 can be easily processed by heating with converting rays, sections 5 and channels between sections 5 can also be formed easily. By using converging rays to form a channel connecting each section 5 during the cultivation of cells, a plurality of different types of cells to be inserted in section 5s can be made to have mutual interaction at a desired time period.

If the space between the opposing substrates 1 and 1' to which electrodes are mounted is regarded as 0.1 mm, the region of sections 5, which is the space between the two substrates to which electrodes are mounted, is almost 0.03×0.03×0.1 mm. If buffer or culture solution, followed by one cell 7 at a time, are inserted into section 5, one cell will be in the space between these electrodes. As the first example of Fig. 1, an example of a cell component sorting chip for floating cells is shown. Here, neutron stem cells are removed one at a time from floating neutron cells, in other words, from a suspension in which neutron stem cells are suspended, inserted into sections 5, and sandwiched between two substrates (substrate 1, substrate 1'). So that neutron stem cells do not implant, agarose film 9, which has a thickness of 0.5 µm or less and a concentration of 0.05% or less, is also applied at the base of each section 5, to prevent cell absorption. This sort of thin agarose film allows free transmission of mRNA, etc. Sections 5 are connected by channel 6' as necessary. As in Fig 1(B), which is a cross-sectional diagram, an agarose gel film 4 dividing the space between substrates 1 and 1' is formed, making each section 5 an individual space, thereby creating a structure in which floating cells are retained in space 5. The structure of the two substrates (substrate 1 and substrate 1'), allows transmission of a 350 nm laser. High 350 nm light absorption at the electrodes is overcome through the use of lattice-shaped electrodes or a metal thin film with a thickness of 5 nm or less, which allows partial transmission of light.

As the second example of Fig. 1, cells are removed one at a time from a suspension in which human COS cells 7 are suspended in PBS at a concentration of 10⁵ cells/ml, inserted into two sections 5, and sandwiched between 2 substrates (substrate 1 and substrate 1'). Since COS cells 7 will implant where there is no agarose gel film 4, (C) is a cross-section showing COS cell 7 in section 5 on substrate 1. For this reason, even if, as shown in (C), agarose gel film 4' is thin and the sections are not completely divided, the cells will not move to the adjacent section, nor will mutual interaction occur between cells outside of the channel. Sections 5 are connected by channel 6' as necessary. Of course, as in Fig 1(B), which is a cross-sectional diagram, an agarose gel film 4 dividing the space between substrates. 1 and 1' may be formed, making each section 5 an independent space, thereby creating a structure in which cells are retained in space 5. In such a situation, each section serves as tunnel 6 instead of the channel.

The structure of the two substrates (substrate 1 and substrate 1'), allows transmission of a 350 nm laser. High 350 nm light absorption at the electrodes is overcome through the use of lattice-shaped electrodes or a metal thin film with a thickness of 5 nm or less, which allows partial transmission of light.

In Fig. 1, spacer 8 is shown entirely surrounding the region provided by section 5. However, in the example in Fig. 1(B), spacer 8 may be arranged so that substrate 1' does not significantly compress agarose gel film 4 and in the example shown in Fig. 1(C), spacer 8 may be intermittently arranged in order to maintain an adequate distance between substrates 1 and 1'. In this instance, the distance between substrates 1 and 1' will be slight, so there will be no culture solution runoff, even in the example shown in Fig. 1 (C).

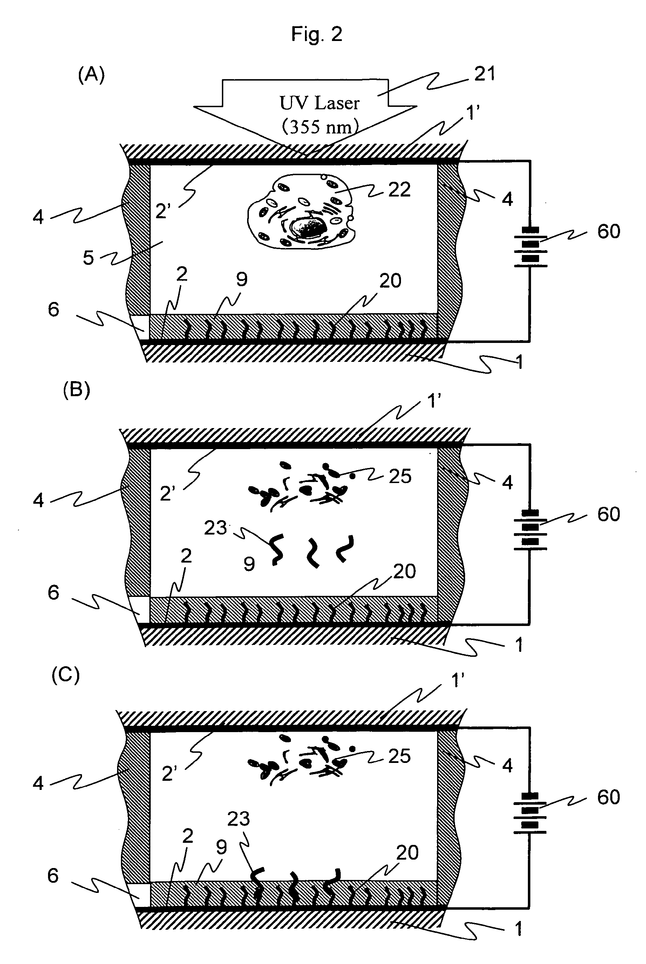

Fig. 2(A), which corresponds to the first example of Fig. 1, shows a cross-section of a cell inside a section 5 sandwiched between 2 substrates used for floating cells (substrates 1 and 1'). A case in which a nonimplanting cell is used is explained hereinbelow. Cell 22 is enclosed in a space divided by substrates 1 and 1' and agarose gel film 4. In this example, agarose gel film 4 is 0.1 mm thick, and the gap between substrates 1 and 1' is also 0.1 mm. Each section 5 is connected by channel 6, which enables intercellular interaction and is prepared arbitrarily during cultivation. Channel 6 has a cross-sectional area that does not allow cell passage. On the inner walls of the 2 opposing substrates, electrode 2 is immobilized to the top of substrate 1, and probe 20s are immobilized to electrode 2. Although probe 20 is actually embedded in agarose gel film 9, since the agarose concentration is thin, mRNA, cDNA, etc. can access the probes freely. Here, probe 20 consisting of polyT is used. Electrodes 2' exist on the surface of substrate 1'. Sections 5 are divided by agarose structure 4, but are joined by tunnel 6 when necessary. Cell 22 is inserted into sections 5 when buffer and culture solution are added therein. Cell 22 is destroyed if 355 pulse laser 21 is used to irradiate section 5, which contains cell 22, from above glass substrate 1'. Electrodes 2 and 2' are for the application of an electrical field and can therefore be located on the back side of a substrate.

Fig. 2(B) is a diagram schematically showing the process in which pulse laser 21 destroys the cells within the section 5s and an electrical charge (1.2 V) is simultaneously applied by power source 60 between electrodes 2 and 2', pulling nucleic acid 23 such as mRNA, DNA, etc. towards electrode 2. Much cellular content such as protein 25 either migrates to minus electrode 2' or remains in its original position without migrating. A pH gradient is formed when an electrical field is applied to the space between electrodes. Although nucleic acids with low pK further migrate to an anode, the electrical charge of other substances having a moderately low pK is neutralized, so those substances can therefore not reach an anode.

Fig. 2(C) is a diagram schematically showing the situation in Fig. 2(B) after the passage of time. As time passes, nucleic acid 23 of mRNA, DNA, etc. hybridizes to polyT probe 20 on the substrate.

Fig. 3 is an example of a human COS cell, which is the cell implanted on substrate 1. This example corresponds to the second example in Fig. 1. Fig. 3(A) is a cross-section diagram showing a cell implanted in section 5, which is sandwiched between, 2 substrates (substrate 1 and substrate 1'). Cell 22' is trapped in the space divided by substrates 1 and 1' and agarose gel film walls 4-1 and 4-2. Since the cell is implanted at the base of the section, the cell will not enter the adjacent section, even if the top of the section is open. The cell cannot implant on parts to which an agarose gel film has been substantially applied and can therefore not migrate to the adjacent section. A substantial agarose thickness of 0.5 µm is adequate to prevent this kind of cell migration. In this example, agarose gel film thickness is 3µm, and substrates 1 and 1' are 0.1 mm apart. Each section 5 is connected by channel 6-1, which enables intercellular interaction and is prepared arbitrarily during cultivation. Channel 6-1 has a width that will not permit the cell to pass through. On the inner walls of the 2 opposing substrates, electrode 2 is immobilized to the top of substrate 1, and probes 20 are immobilized to electrode 2. Here, probe 20 consisting of polyT is used. Electrode 2' exists on the surface of substrate 1'. Sections 5 are divided by agarose structure 4, but are joined by channel 6 when necessary. Cell 22 is inserted into sections 5 when buffer and culture solution are added therein. Cell 22 is destroyed if 355nm pulse laser 21 is used to irradiate section 5, which contains cell 22, from above glass substrate 1'. Electrodes 2 and 2' are for the application of an electrical field and can therefore be located on the back side of a substrate.

Fig. 3(B) is a diagram schematically showing the process in which pulse laser 21 destroys the cells within the section 5s and power source 60 simultaneously applies an electrical charge (1.2 V) between electrodes 2 and 2' in the same manner as in Fig. 2. Although nucleic acid components 23 such as mRNA stop close to the bottom surface of the substrate to which the cell is implanted, the majority of cell content such as protein 25 migrates to minus electrode 2'. A pH gradient is formed when an electrical field is applied to the space between electrodes. Although nucleic acids with low pK further migrate to an anode and hybridize with the probes (polyT) on the substrate, the electrical charge of other substances having a moderately low pK is neutralized, so those substances can therefore not reach an anode.

Fig. 3(C) is a diagram schematically showing the situation in Fig. 3(B) after the passage of time. As time passes, nucleic acid 23 of mRNA, DNA, etc. hybridizes to polyT probe 20 on the substrate.

The following is an explanation of the probe immobilization method. The substrate which is attached on the electrode to which PolyT probes 20 are immobilized or the substrate which is attached on ordinary electrodes is prepared to have a polymer layer by polymerizing 2 types of monomer. The polymer layer has a polymerized structure wherein the first monomer, which has the first polymerization functional group in two locations, and the second monomer, which has the second polymerization functional group in two locations, are arranged alternately and either the first monomer or the second monomer has a biological substance trapping residue. Specifically, an alkyl diisocyanate as the first monomer and a diaminophenylacetate as the second monomer in an equimolar ratio are allowed to react to polymerize. In this case, the first functional group will be an isocyanate group and the second group will be an amino group. Hexamethylene diisocyanate, for example, can be used as an alkyl diisocyanate. In addition, the biological substance trapping residue will be a carboxyl group of diaminophenylacetate, which is the second monomer. In this instance, the polymerization product thin layer is polyurea. Regarding conditions for polymerization, a polymer thin film can be formed on the substrate by applying the above-mentioned mixed solution of 2 types of monomer with a spin coater and then baking the substrate at a predetermined temperature (150°C to 200°C). Alternatively, the known vacuum based deposition method (Jpn. J. Appl. Phys. 33, L1721-L1724 (1994)) can also be used. The polyurea thin film thusly formed on the substrate has a structure formed through alternate reaction of alkyl diisocyanates and diaminophenyl acetate monomers. Accordingly, the calboxylic acid groups in the side chain will exist in a comparatively uniform interval. The interval of the carboxyl group to be introduced can be adjusted by adjusting the length of the alkyl group. In addition, by so doing, thin films having a functional group can be formed even on electrodes of gold, platinum, etc. onto whose surfaces it was conventionally difficult to introduce functional groups. Using a carbodiimide system activator, a biological substance trapping probe having an amino group can be directly immobilized to a carboxyl group introduced onto the substrate. Alternatively, if ethylenediamine is reacted with this side chain carboxyl group in the presence of a dicyclohexylcarbodiimide system activator, a primary amine residue can be introduced into a side chain carboxyl group at a high yield. In other words, the primary amines will be lined up in an approximately uniform interval on the substrate surface. Next, the primary amines are modified with a 1, 4-phenylene diisothiocyanate described in Nucleic Acids Research, 27, 1970-1977 (1999) and CHEMBIOCHEM 2, p. 686-694 (2001) and an isothiocyanate group is introduced at the surface. Finally, the primary amines are reacted with polyT (T₂₅) having an amino group at its 5' terminal. This reaction condition is detailed in the above-mentioned documents. Using the above-mentioned method, a substrate to which polyT is immobilized in an approximate 9 nm interval can be obtained.

Alternatively, if the two monomer types that are polymerized are alkyl diisocyanate with a side chain having a residue of an SH group protected by an alkyl group as the first functional group and diaminophenylacetate with a carboxyl group as the second functional group, the result will be a substrate with a polymer thin film having different, alternating functional groups. Here, a protected SH group, which is an SH group into which a protective group of the SH group used during peptide synthesis has been introduced can be used. Alternatively, the SH group can be protected in the form of a pyridyldithio group. The following explains cases in which monomers with SH groups protected in the form of a pyridyldithio group are used to prepare a substrate. Once the polymer is formed on the substrate, first, ethylenediamine is reacted with a carboxyl group in the presence of a dicyclohexylcarbodiimide system activator as in the above-mentioned method. Next, 1, 4 phenylene diisothiocyanate is reacted, the first oligomer of a specified sequence having an amino group at the 5' terminal is reacted, and the oligomer is immobilized. Next, dithiothreitol is used to reduce the SS bond, and the SH group is exposed to the alkyl group part on the substrate. A second oligonucleotide having a malemide group is reacted in an aqueous solution with an approximate pH of 6. Thus, the double-bonded part of malemide reacts with the SH group on the substrate and the second oligonucleotide can be immobilized with a thioether bond. The merit of this example is that it can be used to obtain a single substrate on which a plurality of probe DNA is introduced at an almost uniform interval. Thus, mRNA with 2 types of complimentary sequence can be trapped on a single surface. Gold nanoparticles having different particle diameters may be used to distinguish between the 2 types of mRNA.

In Example 1, a polyurea having a carboxyl group was formed on a substrate and used. However, a polymer can also be used if the polymer periodically has a residue into which a reaction group that can immobilize polynucleotides, peptides, etc. that are probes in a side chain can be introduced. For example, a combination of two types of monomer can be used wherein the monomers are an acid anhydride having the structure R₁-C=O-O-O=C-R₂-R₃-C=O-O-O=C-R₄ (R₁ to R₄ are arbitrary residues) and a diamine monomer having the structure NH₂-R₅-NH₂ (R₅ is an arbitrary residue) where R₅ is a diaminophenylacetate monomer having a carboxyl group. An acid hydride with a structure such as 4,4'-(hexafluoroisopropylidene)-bis-(phthalic anhydride) may be used. This type of polymer is, namely, a polyimide. This kind of bisphthalic anhydrice and bisamino compound are generally used polyimides. Alternatively, for example, a polyamide that forms a film on a substrate through evaporation polymerization of a diaminophenylacetate having a long carboxyl group or a monomer having a carboxylate residue in the aliphatic position of an aliphatic diamine such as 1, 10-diaminodecane and terephthaloyl dichloride, which is a diacidchloride monomer, can be used. It is sufficient if various reactive groups are introduced into a long chain aliphatic diamine for the purpose of probe immobilization. It is sufficient if evaporation polymerization is carried out according to preexisting methods, for example the methods described in Jpn. J. Appl. Phys. 33, L1721-L1724 (1994) and Thin Solid Films, 215, 94-97 (1992), and in consideration of the vaporization conditions of the monomer in use.

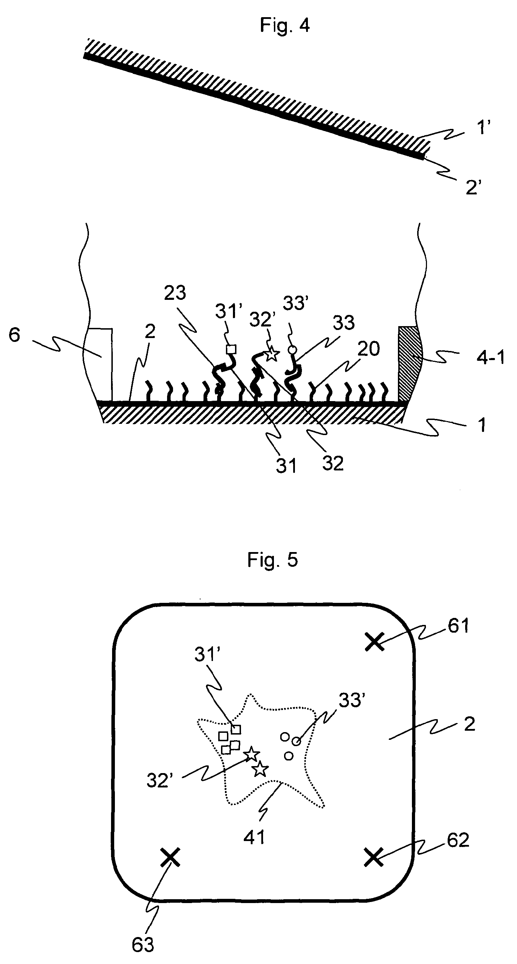

Fig. 4 explains the method for identifying the mRNA groups trapped in polyT probe 20 on the surface of electrode 2 as shown in Fig. 3(C). 3(C) is an example of a cell component sorting chip using an implanting cell. However, as stated previously, if probe 20 is immobilized by being embedded in an agarose gel film 9 with low agarose concentration, mRNA and cDNA can access probe 20 freely, so the below procedure and explanation also apply to the structure described in Fig. 2(C), which is for free-floating cells.

First, upper glass substrate 1' is removed, then the inside of section 5s that formed on the agarose gel film on the upper surface of quartz substrate 1 are washed, then the matter that did not hybridize to polyT probe 20 is removed from electrode 2. Next, since there are different types of mRNA 23, which is hybridized to polyT probe 20, a mixed solution of labeling probes is added to facilitate identification. After incubation at 45°C for 3 hours, washing is performed with a pH 7 citrate buffer solution containing 10mM of NaCl. Under such circumstances, a three-part bond exists on electrode 2 in which the mRNA groups are hybridized to polyT probes 20 and labeling probes 31, 32, and 33 are hybridized to each mRNA. Labeling substances 31', 32' and 33' are attached to labeling probes 31, 32, and 33 in advance so that labeling probes 31, 32, and 33 can be easily identified. Accordingly, the types and quantity of mRNA immobilized to polyT probes 20 on electrode 2 can be measured by counting labeling substances 31', 32' and 33'

Thus, as stated previously, since intracellular substances retain position information while they are immobilized to 2-dimensional surfaces on a substrate or electrode, it is not possible to immobilize a different probe in each section element or to identify the target biological substance based on the site of the element, which is possible with a DNA chip or protein chip. Accordingly, mRNA immobilized to a 2-dimensional surface must be identified by a labeling substance-attached probe hybridized thereto.

Fig. 5 is a diagram schematically showing labeling substances 31', 32' and 33' of the mRNA immobilized to probe 20 on electrode 2 can be recognized on a two-dimensional surface in relation to the outline 41 of the cell 22. 61-63 are markers attached to electrode 2 (or substrate 10). Data regarding the state in Fig. 5 are obtained by the following procedure:
(1) Cells are arranged in sections 5 formed on the agarose gel film 4 on the upper surface of quartz substrate 1, and incubated as required;
(2) Following incubation, cell 22 is viewed from the back of electrode 2 and photographed with a microscope-equipped CCD camera;
(3) A 355 nm pulse laser 21 is used to irradiate section 5, in which cell 22 exists;
(4) After being irradiated with pulse laser 21, an electrical field is applied between electrodes 2 and 2' via power source 60;
(5) After the procedure of washing and labeling probe immobilization explained by Fig. 4, a microscope-equipped CCD camera is used to take another photograph is taken of the back of electrode 2.

In other words, data on cell 22 outline 41 and markers 61 to 63 are obtained in the first shot and saved to a computer as image data. Next, since data on mRNA labeling substances 31', 32', and 33' attached to probe 22 and markers 61 to 63 are obtained, the foregoing are saved to a computer as image data. If two datum are image processed on a computer so that markers line up and are superimposed, image data is obtained in which the relative position of outline 41 of cell 22 and mRNA (labeling substances 31', 32', and 33') can be understood.

An mRNA from a single cell is immobilized atop the substrate by the above-mentioned method in a two-dimensional projection form, the sequence that straddles the portion of the final exon of EpCAM mRNA expressed in epithelial cancer cells (large intestine-derived) and the next-to-final exon, and the sequence corresponding to the intron section of the EpCAM gene genome, and in the same way, the sequence of the portion that straddles the next-to-final exon of the mRNA of CD 44 and the sequence of the intron portion of the CD44 genome are used to design labeled probes, and distribution and quantitative measurement of the foregoing inside a single cell is attempted. EpCAM mRNA probes and EpCAM gene genome intron section probes are bonded to 1.0 nm and 50 nm gold nanoparticles, respectively, while CD44 mRNA probes and CD44 genome intron section probes are bonded to 20 nm and 70 nm gold nanoparticles, respectively, as indexing markers (labeled substances). Particles with a particle diameter distribution within CV=2% are used.

PolyT probes for trapping mRNA are immobilized on electrode 2, cell 22 in section 5 is irradiated with 355 nm pulse laser 21 and then destroyed, and all mRNA is immobilized on electrode 2 by the above-mentioned method in which an electrical field is used to trap mRNA in the polyT probes. In the above situation, intracellular position information can be retained because intracellular mRNA is immobilized to the substrate as if projected thereon. Cell 22 is implanted on electrode 2 by arranging the above-mentioned epithelial cell in section 5 and then incubating said cell for 1 day in a medium supplied to section 5 in a 5% carbon dioxide, 11% oxygen atmosphere. The position of the immobilized cell is confirmed in advance with a microscope, the back of electrode 2 is photographed through a microscope-equipped CCD camera, and the image is converted to data. When in this state, the cell is contacting the following surfaces: a) the surface of electrode 2, which said cell is contacting; and b) the surface contacting the medium. In the present invention, data are obtained by projecting mRNA, etc. two-dimensionally. mRNA, etc. are projected onto the surface of the substrate that makes contact with the cell immobilized thereon. Thus, the intracellular functions of the cell surface bonded to the substrate and the cell surface contacting the solution differ. Useful information can be obtained from two-dimensional as well as three-dimensional projections because the electrode surface making contact with the cell can always be used as the standard surface when analyzing the distribution of intracellular substances.

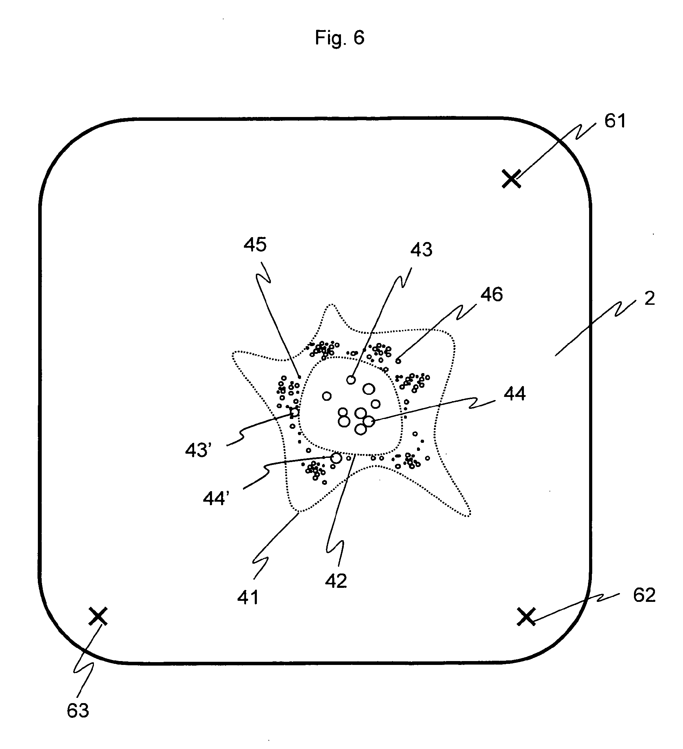

A suspension, comprising labeling probes 31, 32, and 33, which are probes for mRNA and a genome sequence of EpCAM and CD44, and which are bonded to indexing markers 31', 32', and 33', which are gold nanoparticles, is added to each section 5 in which polynucleotide 23 such as mRNA and genome DNA were captured on electrode 2 so as to induce hybridization. As a condition for efficient hybridization without coagulation of the gold nanoparticles, hybridization is performed with pH 7.4 citrate buffer solution containing 100mM of NaCl. Consequently, results such as those obtained in Fig. 6, a schematic diagram, can be obtained. The drawing is altered so that position information for each index marker (gold nanoparticle) of the cell can be discerned.

The large intestinal cancer-derived cancer cell implanted in section 5 for cell retention that was formed by agarose gel film 4 on substrate 4 is observed in advance with a microscope. The boundary of the cell when it is converted into data by the CCD camera is 41, while 42 is the boundary of the nucleus. Cells are destroyed with 355 nm pulse laser 21 and the electrical field created by the voltage applied between electrodes 2 and 2' immobilizes polynucleotide components atop electrodes 2. On the other hand, if the top of electrode 2 is observed with a scanning electron microscope and converted into data here, the distribution of the sizes of the 4 types of gold nanoparticles 43 to 46 can be observed. Judging by the size differences, it is thought that 43 detects polynucleotides having sequences corresponding to EpCAM gene intron while 44 detects polynucleotides having sequences corresponding to the CD44 gene. These are mostly detected in nuclei. It can be considered that intron sequences detected in nuclei are derived from immature mRNA transcribed from genome sequences and genomes. Although a small quantity of particles 43' and 44' which recognize intron sequences are detected in the cell chamber, these can be thought of as intron sequences clipped from immature mRNA before the intron was broken down. Gold nanoparticles 45 and 46 exist not in the nuclei, but in the cell chamber and can be thought of as mature mRNA for EpCAM and CD44, respectively. This is because introns sometimes fall off of mature mRNA.

In the present invention, a gene sequence on a genome, immature mRNA, or mature mRNA can thusly be detected on the basis of individual molecules.

### [EXAMPLE 2]

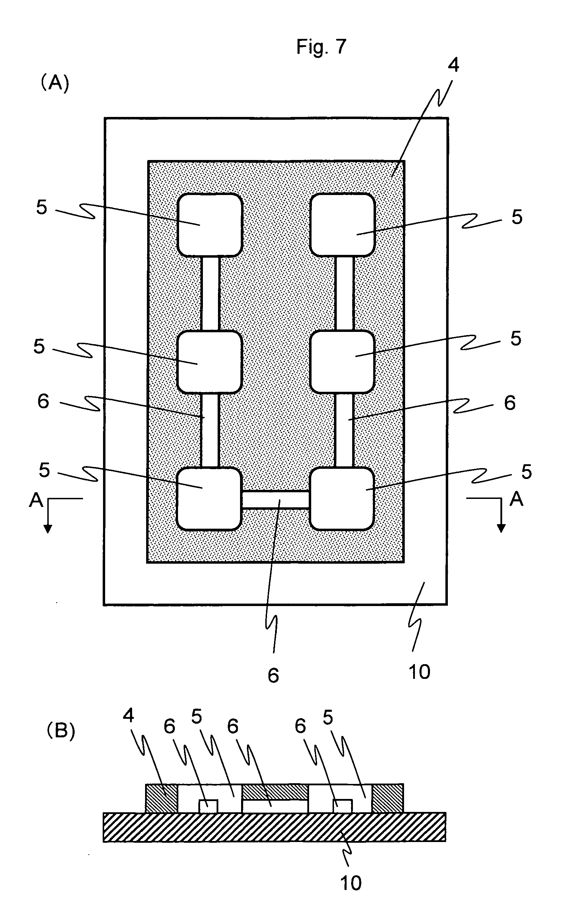

In Example 2, a method is used in which cells are instantaneously burned onto a substrate without cell destruction via a laser or cell component migration via electrolysis. Fig. 7(A) is a plan view showing the cell component sorting chip of Example 2. Fig. 7 (B) is a cross-sectional diagram showing the view from the direction of the arrows in the A-A position of Fig. 7(A). 10 is a substrate. In Example 2, two types of alternating monomer are polymerized, and a substrate having alternating, positively-charged amino groups and hydrophobic groups is used instead of quartz to facilitate simultaneous adsorption immobilizing of mRNA and expressed proteins. For example, 4,4'-methylenediphenyl diisocyanate with a benzene ring as a hydrophobic residue as the first monomer and the diaminophenylacetic acid shown in Example 1 as the second monomer are reacted in an equimolar ratio and polymerized. Alternatively, a polymer wherein the diaminophenylacetic acid and 4, 4'-hexafluoroisopropylidenebis(phthalic anhydride) are polymerized and hydrophilic groups and hydrophobic groups are alternately introduced can be used.

In this state, there is a hydrophobic residue but not positively-charged residue on the substrate surface, so the carboxyl group of the diaminophenylacetic acid is exchanged with an amino group. Ethylenediamine is reacted with a carboxyl group in the presence of a dicyclohexylcarbodiimide system, and a carboxyl group is transformed into a primary amine residue (amino group). Due to this transformation, a substrate having a surface comprising a polymer thin film alternately having a hydrophobic residue and an amino group can be obtained. Although a substrate having a polymer thin film alternately having a hydrophobic residue and an amino group was used here, a thin film in which nylon, a polyamide has been polymerized on the substrate or a substrate having a polystyrene thin film can also be used.

On the thusly prepared substrate 10, as in Example 1, a 100 µm-thick agarose gel film 4 is formed and sections 5, which retain cells, are prepared at a predetermined interval. Section size is, for example, 30µm square. If necessary, each section 5 is connected by a channel 6, making mutual interaction possible between cells 7 that are retained in sections 5s possible. In Example 2, there is no electrode 2 or upper surface glass 1'.

As in Example 1, buffer or culture solution is inserted into section 5, the cell is arranged, and the cell is incubated according to predetermined conditions if necessary. After water is gently shaken from substrate 10, upon which the cell is implanted, is dried at 160°C in a helium atmosphere. The cell is thereby dried and adheres to the surface of substrate 10 instantaneously. Alternatively, substrate 10, upon which the cell is implanted, can be instantaneously frozen in liquid nitrogen and then dried. Alternatively, cells can also be fixed to the substrate surface through the use of reagents such as formaldehyde and glutaraldehyde. The intracellular mRNA and proteins can thereby fix to the substrate while retaining intracellular two-dimensional information. Next, the substrate is immersed in 70% alcohol and the low-molecular weight compounds that did not immobilize thereon are removed.

Since the amine residue of a polynucleotide or the amine group of a protein is modified when formaldehyde or glutaraldehyde is used, said amine residue and amine group are exposed to water vapor at 100°C and restored when necessary.

In order to hybridize the labeling probes to the mRNA inside the cytoskeleton, the cell membrane is processed so that the labeling probes can react thereto. Generally-used treatment with enzymes is used here. For example, a solution containing pepsin is added, holes are opened in the cell membrane surface, and washing is performed with PBS (pH 7.4) so that the probe labeling substance will react with mRNA.

Indexing probes in which 10 nm gold particles are bonded to probes for an EpCAM mRNA sequence and indexing probes in which 20 nm gold particles are bonded to RNA aptamers for EpCAM proteins are used, thereby simultaneously detecting EpCAM mRNA and protein.

An RNA aptamer composite with an affinity to EpCAM is prepared through the use of an evolutionary engineering method. The method is explained hereinbelow. An RNA aptamer that bonds to EpCAM is prepared as follows: as shown in SEQ ID NO:2, a sequence with a 26 base length containing a T7 promoter is introduced at the 5' terminal of a single strand of DNA comprising a random sequence of 40 bases. Then a priming site comprising 23 bases is inserted at the 3' terminal of the above-mentioned single strand of DNA to yield a sequence with a total length of 89 bases. The sequence is, from the 5' terminal, TAATACGACTCACTATAGGGAGACAA(40)TTCGACAGGAGGCTCACAACA GG: (SEQ ID NO: 2). The T7 promoter sequence is used with the RNA polymerase to perform RNA transcription. In RNA transcription, 100 u of T7 polymerase is applied to 100 pmol of DNA at a 500µl scale. The substrates are 3 mM of 2'-F-CTP and 2'-F-UTP and 1 mM of ATP and GTP. The foregoing are used to perform transcription at 25°C for 10 hours. After RNA transcription, DNA is degraded with DNaseI and transcription the RNA product is recovered through electrophoresis. After the recovered transcription RNA is heat denatured, it is passed through an EpCAM-immobilized sepharose CL4B column in PBS (pH 7.4) containing 2 mM of MgCl₂ A bonded transcription RNA component is eluted with solution containing 7M urea. The obtained transcription RNA component is reverse transcripted and then PCR amplified by a primer pair of a sequence that is complimentary to the known sequence section at both ends. The obtained PCR product is transcripted again with the T7 promoter, trapped in the EpCAM immobilizing sepharose CL4B column in the same way as above, and the bonded transcription RNA components are recovered. The process of transcription-trapping-recovery-PCR amplification is repeated 15 times, and an RNA aptamer that specifically reacts to EpCAM is obtained.

A thiophosphoric acid group is inserted at terminal 5' of the thusly obtained aptamer by means of in vitro transcription according to the method described in the article "Staining of cell surface human CD4 with 3'-F-pyrimidine-containing RNA aptamers for flow cytometry" in Nucleic Acids Research 26, 3915-3924 (1998). Biotin in which an iodoacetyl group was introduced at the thiophosphoric acid group is reacted and a 5'biotinylated RNA aptamer is obtained. The streptavidin conjugate gold nanobeads are reacted and an RNA apatamer that reacts specifically to EpCAM with gold nanoparticles as the substance being identified is obtained.

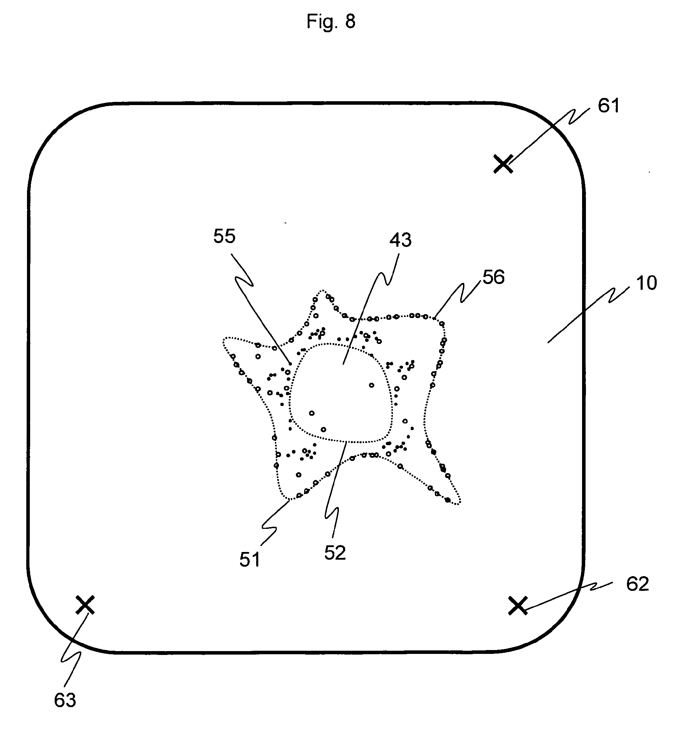

Indexing probes in which 10 nm gold particles are bonded to probes for an EpCAM mRNA sequence and indexing probes in which 20 nm gold particles are bonded to RNA aptamers for EpCAM proteins are mixed in an equimolar ratio and a solution to which a RNase inhibitor has been added is added to the substrate to which cells are immobilized. Incubation is conducted for 2 hours at 45°. Following the completion of the reaction cleaning is performed with chilled water, and observation is conducted with a scanning electron microscope or an atomic force microscope. The thusly obtained results are shown in Fig. 8. As with Fig. 6, which shows the results obtained in Example 1, the position of cell membrane 51 and nucleus membrane 52 are verified before cell immobilization. The gold nanoparticles bonded to EpCAM mRNA in view of particle diameter are shown in 55, while the gold nanoparticles thought to be bonded to EpCAM protein are shown in 56. In this case, it is understood that there is mRNA in the cytoplasm. In addition, although EpCAM protein is entirely detected, since there is a considerable concentration on the boundary of 51, it can be thought that EpCAM protein is localized on the cell surface in this type of case.

In Example 2, genomes are simultaneously immobilized on the substrate in addition to mRNA and proteins. Consequently, by reacting the forgoing with probes having nanoparticles that correspond thereto, quantitative data having spatial information on mRNA and protein expression of the same cells on the same substrate can be obtained. Specific genes of the genome that exists in the nucleus can also be distinguished from the mRNA and simultaneously detected. In order to realize the foregoing, a different nanoparticle should be used for the mRNA, the proteins, and the genomes. For example, nanoparticles with different particle diameters having a gold nanoparticle base and doped with various elements can be used on mRNA, identical particles with a silver base can be used on proteins; and identical particles with a platinum base can be used on genomes. Alternatively, fluorescent nanoparticles or branched probe DNA with fluorescence can also be used for genomes. Measurement is not performed simultaneously. Rather, it is also effective to allow reactions of probes having nanoparticles for mRNA, proteins, and genomes in order, and then perform detection at intervals.

### [EXAMPLE 3]

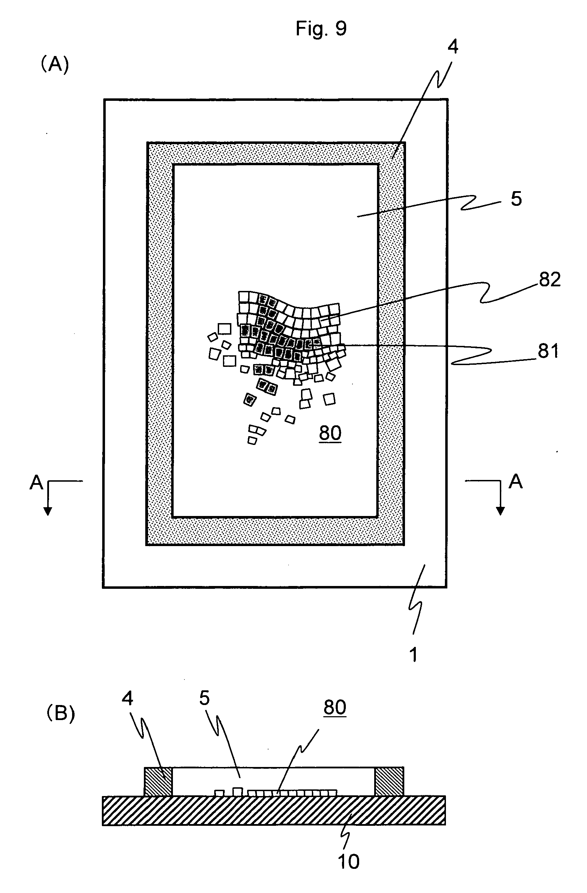

Example 3 is an instance in which a tissue-like specimen of aggregated cells, for example, a histological section is immobilized to a two-dimensional surface while position information of an intracellular substance is retained. Fig. 9(A) is a plan view showing the cell component sorting chip of Example 3. Fig. 9 (B) is a cross-sectional diagram showing the view from the direction of the arrows in the A-A position in Fig. 9(A). Substrate 10 is frozen in advance, tissue is placed thereon, and a single cell layer is frozen and immobilized, peeled off, and thereby prepared. Alternatively, microtome can be used to arrange the frozen cell structure in each section 5 of substrate 10 as a 2 µm-thick slice. In Example 3, a tissue-like specimen of aggregated cells is the target. Therefore, instead of providing a section 5 for each cell as in Examples 1 and 2, section 5 is made to store the tissue-like specimen. In other aspects, the form of either Example 1 or Example 2 is acceptable, however, the form in Fig. 9 is identical to that of Example 2. In other words, indexing probes in which 10 nm gold particles are bonded to probes for an EpCAM mRNA sequence and indexing probes in which 20 nm gold particles are bonded to RNA aptamers that bond to EpCAM proteins are used and EpCAM mRNA and protein are simultaneously detected. When an attempt is made to detect the EpCAM mRNA 10 nm gold particles thought to be bonded to mRNA derived from EpCAM at specific cell layer 81 of tissue fragment 80 and 20 nm gold particles thought to be bonded to EpCAM protein are detected. Results are obtained wherein the number of undetected or detected gold nanoparticles from other section 82 are 1/100 or below in comparison with those in cell position 81.

In the present invention, cells are observed as projected onto a two-dimensional surface, and multiple cells will overlap, making a single layer preferable. If this is two or more layers, the cell components of each layer will overlap and the anticipated information will not be obtained.

### [EXAMPLE 4]

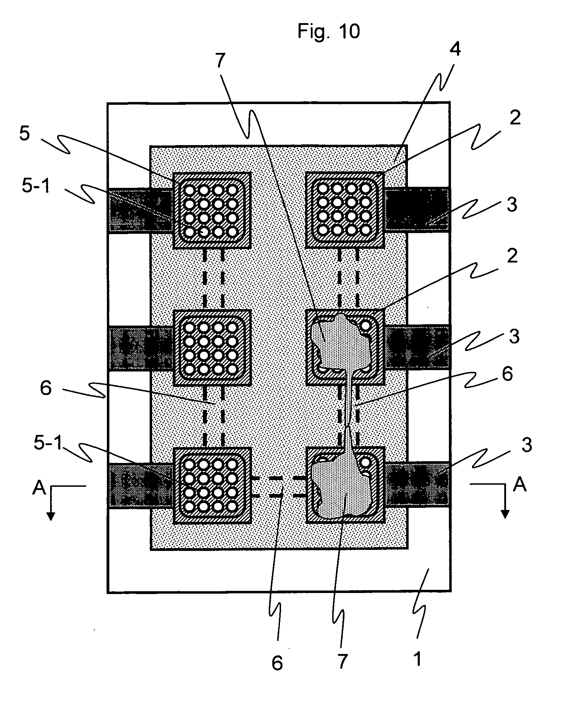

In Example 4, as in Example 1, the cell component sorting chip shown in Fig. 10, which can retain a cell in each section while maintaining interaction between multiple cells, is used. This explanation will focus on differences to Figure 1. Different probe 5-1s are arranged in the form of spots in the region of section 5 (almost 0.05 mm × 0.05 mm). Here, a system is explained wherein probes are prepared in a total of 16 points at a 10 µm pitch to obtain spots that are approximately 5 µm. By using an inkjet, spot diameter can be reduced to roughly 1 µm, making it possible to prepare spots at a 2µm pitch and prepare a chip capable of simultaneously measuring roughly 500 items. Moreover, this chip can measure a plurality of cells individually. First, an intercellular network is prepared by arranging cell 7 in each section 5 and cultivating cell 7 for a predetermined period of time, thereby forming an artificial tissue on which cells are arranged atop the chip. Member 4, which divides each cell section, is made of agarose gel. After arranging the cells, intersectional space is heated with a laser to remove the agarose gel therefrom, thereby forming tunnel 6, causing the arbitrary cells to interact in the arbitrary order. It is also possible, of course, to prepare the cell retaining sections from a light-cured resin such as SU8. In this instance, fluid channel 6 is prepared in advance.

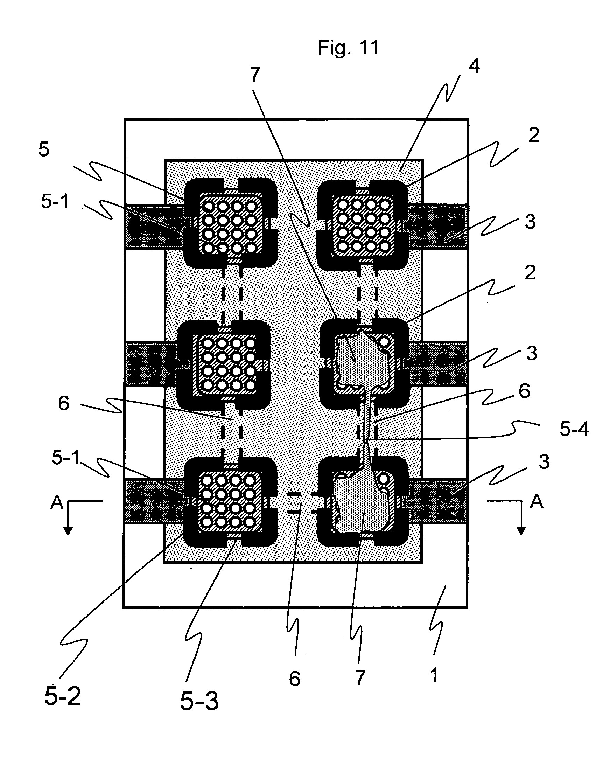

In the step described hereafter, the most suitable structure to prevent spreading of the biological substance as much as possible is, as shown in Figure 11, a chip in which SU8 section 5-2 is prepared in advance, and there is an agarose gel thin film 4 between each section. SU8 section5-2 has open slits 5-3 in four directions. If channel 6, from which agarose is removed, is prepared between the sections 5-2, cells 7 arranged at each section can use slits 5-3 and channel 6 to mutually form junction 7-1.

Next, UV light is used to destroy cells. In this state, the biological substance is especially prone to spreading if immobilizing operations are not performed thereon within member section 5-2, which is substantially divided by SU8. The SU8 divider can minimize the leakage of the biological substance outside the section. Either incubation is performed for a set period of time or the predetermined voltage is applied to electrode 2 as shown in Example 1 and an affinity bond between the biological substance arranged within sections 5 and probe 5-1 is formed. In other words, the substances that correspond to the 16 items of probes are trapped in the spot position on the substrate. The trapped substance is further taxonomically labeled with a nanoparticles-labeled probe and the particles are counted with a scanning electron microscope or an atomic force microscope.

For example, three types of capture probe are immobilized to each spot as a mixture, and detection DNA probes are used that are labeled with nanoparticles having 10 types of different particle diameter and different material combinations, and that correspond respectively to each trapping probe. In other words, there are 16 × 10 types of detection DNA probes, in a mixture solution of DNA probes labeled by nanoparticles with 10 types of particle diameter differing for each 16 types. Particles with diameters of 10, 20, 30, 40, and 50 nm are used. As materials, if gold and silver are used, there are a total of 10 types of label substance.

For example, as in the above examples, destroy the cell in sections 5 with UV light, incubate the mRNA for hybridization for 2 hours, then add a mixture solution of the nanoparticle-labeled DNA probes and incubate for 2 hours for hybridization with the mRNA groups captured on the substrate surface. After washing and drying, if the nanoparticles bonded to the surface of each section 5 are comprehensively scanned with an electron scanning microscope, the quantity of 10 types of nanoparticles on each spot can be counted. As a result, quantitative analysis of the 160 items of mRNAs per cell becomes possible.

Systems that use aptamer for intracellularly synthesized proteins and glycoproteins as a substance immobilized to spots are explained here. In this Example, DNA aptamers are used as the aptamers for proteins and glycoproteins to be individually measured. DNA aptamer is prepared according to the SELEX method in the prior art (J. Clin. Invest. 98, 2688-2692 (1996), Nucleic Acids Res. 24, 702-704 (1996)). Each aptamer is spotted onto all of the section 5s that are divided by SU8 structure 5-2. Since all aptamers have an amino group introduced at the 5' terminal, the method described in Example 1 is used to immobilize the amino group to the substrate. The aptamer for CD62L that is described in J. Clin. Invest. 98, 2688-2692 (1996) and the aptamer for thrombin that is described in Nucleic Acids Res. 24, 702-706 (1996), for example, are immobilized on the substrate. The thusly prepared aptamer spot array (5-1, Fig. 11) is inserted into cell 7 of each section 5 of chip 1 which corresponds to a plurality of cells all of which have a plurality of section 5s, and gap junction 5-4 is formed between sections, thereby forming a cell network. When cells are destroyed with a UV laser and incubated for 30 minutes, proteins and glycoproteins that correspond to the aptamers are captured in each spot of section 5. Next, unreacted biological substance is washed with a solution of PBS (pH 7.4) containing 0.05% of Tween20 and 10 mg/ml of BSA. A mixture of nanoparticle-labeled antibodies of various particle diameters suspended in the same solution is added to the surface of substrate 1 and then reacted for 15 minutes. Each antibody is individually labeled with gold nanoparticles having a different particle diameter for each type. Here, the nanoparticles bonded to the antibodies that correspond to CD62L are 20 nm gold nanoparticles. After the reaction, detecting the existence of gold nanoparticles having the above-mentioned particle diameter will make it possible to measure 20 nm gold nanoparticles in locations where the aptamer for CD62L was spotted. In other spot locations, results are obtained showing that only a background level is measured.

Although the above explains illustrative examples of the present invention, various changes, modifications, and improvements could easily be thought of by one skilled in the art. The above is merely illustrative of the principles of the present invention. One skilled in the art could make various modifications without departing from the scope and spirit of the present invention.

### INDUSTRIAL APPLICABILITY

By the present invention, position information and quantity of intracellularly-expressed mRNA and proteins in addition to genomes bearing the information thereof within and between cells can be detected at the molecular level. The present invention is useful as a cell component sorting chip, as a cell component analysis system, and as a cell component analysis method that make it possible to overcome the above-mentioned problems, and quantitatively measure the mRNA and proteins localized between or inside cells without losing local space data.

## Claims

1. A cell component analysis system comprising:
a substrate structure wherein probes for the capture of a predetermined biological substance are anchored to a substrate surface;
a spatial structure that retains a cell-containing buffer on a surface upon which the probes for the capture of a biological substance of the substrate structure are provided;
a mechanism that destroys cells in the buffer retained in the spatial structure;
a mechanism that enables two-dimensional projection of the predetermined biological substance contained in the destroyed cell onto the substrate surface; and
a mechanism that enables distinction between and detection of biological substances trapped by a probe on the substrate surface.

2. A cell component sorting chip for cell component analysis comprising:
a substrate having a region divided into predetermined sections;
a means that enables communication between the regions of the substrate as necessary;
probes provided on the surface of the region of the substrate, said probes being able to trap the components of a cell contained in said region; and
electrodes that apply an electrical field from above and below the regions of the substrate.

3. The cell component sorting chip according to claim 2, wherein the surface of the divided regions is a polymer layer prepared by the polymerization of two types of monomer, the polymer layer is a structure polymerized such that the first monomer having the first polymerization functional group in two locations and the second monomer having the second polymerization functional group in two locations are arranged alternately, and either the first monomer or the second monomer has a biological substance trapping residue.

4. The cell component sorting chip according to claim 2, wherein the surface of the divided regions is a polymer layer prepared by the polymerization of two types of monomer, the polymer layer is a structure polymerized such that the first monomer having the first polymerization functional group in two locations and the second monomer having the second polymerization functional group in two locations are arranged alternately, and both the first monomer and the second monomer have a biological substance trapping residue.

5. The cell composition substance sorting chip according to claim 3 and claim 4 wherein the polymer layer is formed on the electroconductive member surface provided on the substrate surface.

6. The biological substance analysis system according to claim 1 wherein the mechanism that two-dimensionally projects the destroyed cell containing the predetermined biological substance comprises a power source and electrodes that apply an electrical field on either side of a spatial structure for retention of a buffer containing the cell.

7. The biological substance analysis system according to claim 1 wherein the mechanism that destroys the cells is capable of ultraviolet irradiation of the cells at a wavelength of 320 nm to 400 nm.

8. The biological substance analysis system according to claim 1 wherein the mechanism that enables distinction between and detection of biological substances trapped in the probes on the substrate surface is nanoparticles that label the labeled probe that hybridizes to the biological substance and a measurement means that identifies the nanoparticles wherein the nanoparticles are specified by size and material.

9. The biological substance analysis system according to claim 1 wherein the surface of the substrate to which the biological substance trapping probe is anchored is prepared by the polymerization of two types of monomer, the polymer layer is a structure polymerized such that the first monomer having the first polymerization functional group in two locations and the second monomer having the second polymerization functional group in two locations are arranged alternately, and either the first monomer or the second monomer has a biological substance trapping residue.

10. A cell component sorting method wherein a cell component sorting chip for cell component analysis provided with a substrate having a region divided into predetermined sections, a means that enables communication between the regions of the substrate as necessary, probes provided on the surface of the region of the substrate that can trap the components of a cell contained in said region, and electrodes that apply an electrical field from above and below the regions of the substrate is used, said method comprising the following steps:
retaining a cell-containing buffer solution in a region of the section;
destroying the cell in the region of the section;
two-dimensionally projecting the predetermined biological substance contained in the destroyed cell onto a region of the substrate surface and capturing a predetermined substance in the probes;
reacting probes labeled with predetermined nanoparticles with the captured predetermined substance; and
analyzing the particle diameter and elemental composition of the nanoparticles of the reacted probes to identify the type and position of each molecule of the predetermined biological substance.

11. A cell component sorting chip comprising:
a substrate having a region divided into predetermined sections for retaining individual cells;
multiple types of probes capable of capturing the cell components arranged in spots on the divided region of the substrate; and
a mechanism that enables communication between the regions of the substrate as necessary.

12. A cell component analysis method wherein a cell component sorting chip provided with a substrate having a region divided into predetermined sections for retaining individual cells, multiple types of probes capable of capturing the cell components arranged in spots on the divided region of the substrate and a mechanism that enables communication between the regions of the substrate as necessary, and a cell component analysis chip for cell component analysis provided with electrodes for the application of an electrical field from above and below the regions of the substrate are used, said method comprising the following steps:
retaining a cell-containing buffer solution in a region of the section;
destroying the cells in the region of the section;
capturing the predetermined substance contained in the destroyed cells with the plurality of probes that exist in the regions of the substrate;
reacting a probe labeled with predetermined nanoparticles with the captured predetermined substance; and
analyzing the particle diameter and elemental composition of the nanoparticles of the reacted probe to identify the type and position of each molecule of the predetermined biological substance.

13. A cell component analysis method wherein a cell component sorting chip for cell component analysis provided with a substrate having a region divided into predetermined sections and a means for communicating between the regions of the substrate as necessary is used, said method comprising the following steps:
retaining a cell-containing buffer solution in a region of the section;
drying the cells retained in the section of the region by exposing said cells to a dry atmosphere of predetermined requirements or freezing and then drying the section of the region to which the cells implanted,
adding a predetermined solution to make the cell membrane permeable,
washing substances other than cell components immobilized to the section of the region;
reacting probes labeled with predetermined nanoparticles with cell components immobilized to the section of the region; and
analyzing the particle diameter and elemental composition of the nanoparticles of the reacted probe to identify the type and position of each molecule of the predetermined biological substance.

14. A cell component analysis method wherein a cell component sorting chip for cell component analysis provided with a substrate having a region divided into predetermined sections and a means for communicating between the regions of the substrate as necessary is used, said method comprising the following steps:
retaining a cell-containing buffer solution in a region of the section;
exposing cells trapped in the section of the region to a solution containing aldehydes to immobilize the cell components;
adding a predetermined solution to make the cell membrane permeable,
washing cell components immobilized to the section of the region;
reacting probes labeled with predetermined nanoparticles with cell components immobilized to the section of the region; and
analyzing the particle diameter and elemental composition of the nanoparticles of the reacted probe to identify the type and position of each molecule of the predetermined biological substance.

15. A cell component analysis method wherein a cell component sorting chip for cell component analysis provided with a substrate having a region divided into predetermined sections to which predetermined markers are immobilized, a means for communicating between the regions of the substrate as necessary, probes provided on the surface of the region of the substrate, said probes being able to trap the components of a cell contained in said region, and electrodes for the application of an electrical field from above and below the regions of the substrate is used, said method comprising the following steps:
retaining a cell-containing buffer solution in a region of the section;
obtaining a first image wherein the cells retained in the region of the section and the marker fit into a single screen;
destroying the cells in the region of the section;
two-dimensionally projecting the predetermined biological substance contained in the destroyed cells onto a region of the substrate surface and trapping a predetermined substance in the probes;
reacting probes labeled with predetermined nanoparticles with the captured predetermined substance;
obtaining a second image wherein the probe-labeling nanoparticles and the marker fit into a single screen, and
analyzing the particle diameter and elemental composition of the nanoparticles of the reacted probe to identify the type and position of each molecule of the predetermined biological substance; and
matching the markers of the first image and the second image, and superimposing said images.

16. A cell component analysis method wherein a cell component sorting chip for cell component analysis having a substrate having a region divided into predetermined sections to which predetermined markers are immobilized, said substrate provided with a means for communicating between the regions of the substrate as necessary is used, said method comprising the following steps:
retaining a cell-containing buffer solution in a region of the section;
obtaining a first image wherein the cells retained in the region of the section and the marker fit into a single screen;
drying the cells retained in the section of the region by exposing said cells to a dry atmosphere of predetermined requirements or freezing and then drying the section of the region to which the cells implanted;
adding a solution containing pepsin to make the cell membrane permeable;
washing cell components immobilized to the section of the region;
reacting probes labeled with predetermined nanoparticles with cell components immobilized to the section of the region;
obtaining a second image wherein the probe-labeling nanoparticles and the marker fit into a single screen,
analyzing the particle diameter and elemental composition of the nanoparticles of the reacted probe to identify the type and position of each molecule of the predetermined biological substance; and
matching the markers of the first image and the second image, and superimposing said images.

17. A cell component analysis system comprising:
a substrate structure wherein a probe for the capture of a predetermined biological substance is immobilized to a substrate surface;
a mechanism coated with an agarose thin film for prevention of cell adsorption to the substrate surface upon which the probes are immobilized;
a spatial structure that retains a cell-containing buffer on a surface upon which the probes for the capture of a biological substance of the substrate structure are provided;
a mechanism that destroys cells in the buffer retained in the spatial structure,
a mechanism that enables two-dimensional projection of the predetermined biological substance contained in the destroyed cell onto the substrate surface, and
a mechanism that enables distinguishing and detecting biological substances trapped by a probe on the substrate surface.

18. A cell component sorting chip comprising:
a substrate having one or more sections that contain biological substance trapping probes, wherein the section is defined by (a) the first surface on the substrate that has affinity or non-affinity to the cell, (b) the side walls that have affinity to the cell and are arranged on the first surface, and (c) the second wall facing the first surface via a spacer, and said section has a volume capable of retaining sample solution containing at least a single cell;
the biological substance trapping probes are immobilized to the first surface, the side walls are as high or lower than the spacer, and a pair of electrodes for the application of an electric field is provided the first surface and the second surface.

19. The cell component sorting chip according to claim 18 wherein each of the multiple sections is connected to the neighboring section so as to allow fluid communication.

20. The cell component sorting chip according to claim 18 wherein the pair of electrodes are parallel plate electrodes, the first surface is formed from one surface of one of the electrodes, and the second surface is formed from one surface of the other electrode.

21. The cell component sorting chip according to claim 18 wherein the first surface that has affinity to the cell comprises a polymer layer containing the biological substance trapping probes.

22. The cell component sorting chip according to claim 18 wherein the first surface that has non-affinity to the cell comprises a polymer layer containing the biological substance trapping probes and a layer of agarose gel applied on the polymer layer.

23. The cell component sorting chip according to claim 22 wherein the agarose concentration does not exceed 0.05%.

24. The cell component sorting chip according to claim 21 or claim 22 wherein the polymer layer contains a heteropolymer comprising two types of monomer alternately bonded and the biological substance trapping probes are bonded to one of the two types of monomer.

25. The cell component sorting chip according to claim 18 wherein a plurality of spots to which biological substance trapping probes are immobilized are formed on the first surface of the interior of each section on the substrate.

26. The cell component sorting chip according to claim 18 wherein the side walls that have non-affinity to the cell are formed from agarose gel or light curing resin.

27. The cell component sorting chip according to claim 18 wherein the pair of electrodes are for the application of an electrical field to move the biological substance being tested towards the biological substance trapping probes.

28. The cell component sorting chip according to one of claim 18 to claim 27 wherein the plurality of types of biological substance probes are immobilized to the first surface in accordance with the biological substance being tested.

29. A device for cell component analysis that uses a biological substance sorting chip according to one of claim 18 to claim 28 comprising:
a means for placement of the biological component retaining the test solution containing at least one cell at the interior of the section;
a means for irradiating the interior of the section with UV light having a wavelength absorbed by the cell in order to destroy the cell at the interior of the section and force emission of the cell content that contains the biological substance being tested;
a power source that supplies electric power between the pair of electrodes; and
a means for detecting and/or quantifying a biological substance trapped at the first surface.

30. The device according to claim 29 wherein the detection and/or quantitative means contains an imaging device, electron scanning microscope, or atomic force microscope provided with a CCD camera.

31. A system for the analysis of cell components comprising:
the cell composition sorting chip according to one of claim 18 to claim 28;
a means for irradiating the interior of the section with UV light having a wavelength absorbed by the cell in order to destroy the cell at the interior of the section and force emission of the cell content that contains the biological substance being tested;
a power source that supplies electric power between the pair of electrodes; and
a means for detecting and/or quantifying a biological substance trapped at the first surface.

32. A method of cell component analysis using a biological component sorting chip according to any one of claim 18 to claim 28 comprising the following steps:
(i) injecting sample solution containing at least a single cell into the section;
(ii) irradiating the interior of the section with UV light having a wavelength absorbed by the cell in order to destroy the cell at the interior of the section and force emission of the cell content that contains the biological substance being tested;
(iii) using the net electric charge of the biological substance being tested to apply an electrical field between the first and second surfaces, moving the biological substance emitted from the cell towards the biological substance probes, and trapping the biological substance with the probes;
(iv) washing away cell content other than the biological substance trapped in the probes;
(v) using a labeling substance to label the biological substance trapped in the probes, and
(vi) detecting and/or quantifying the labeled biological substance under a microscope.

33. The cell component analysis method according to claim 32, wherein the plurality of biological substance trapping probes use the cell component sorting chip immobilized to the first surface, the multiple types of biological substance trapped in probes in steps (v) and (vi) are labeled with a different labeling substance according to type, and the different labeled substances are detected and/or quantified and the biological substance being tested is thus identified.

34. A cell component analysis method according to claim 32 wherein a cell component sorting chip having a first surface that has affinity to the cell is used, said method comprising the following steps:
prior to step (ii), recording image (a), which shows the image projected substantially vertically onto the first surface of the cell that is adhered to the first surface that has affinity to the cell;
after step (v), recording image (b), which shows the same range as image (a); and,
in step (vi), obtaining position information regarding various biological substances inside the cells by superimposing image (a) and image (b).

35. A cell component analysis method wherein a cell component sorting chip having a substrate provided with 1 or more sections for the retention of sample solution is used, the section is defined by (a) the first surface which is atop the substrate and has affinity with the cell and (b) the side walls which are arranged atop the first surface and do not have affinity with the cell, and has a volume capable of retaining sample solution containing at least a single cell,
said cell component analysis method comprising the following steps:
(i) injecting sample solution containing at least a single cell into the section;
(ii) dry the sample solution in the section, thereby adhering the cell to the first surface,
(iii) treating the cell with an enzyme to make the cell membrane permeable to the labeled probes;
(iv) using labeled probes that specifically bond with the biological substance being tested to label the biological substance in the cell; and
(v) detecting and/or quantifying the labeled biological substance.

36. The method according to claim 35 wherein the first surface contains a polymer layer having a positive charge.

37. A method according to claim 35 wherein step (ii) comprises exposing the sample solution to a dry helium atmosphere or freeze drying the sample solution with liquid nitrogen.

38. The cell component analysis method according to claim 35 comprising the following steps:
following step (ii), recording image (a), which shows the image projected substantially vertically onto the first surface of the adhered cell;
after step (iv), recording image (b), which shows the same range as image (a); and,
in step (v), obtaining position information regarding various biological substances inside the cells by superimposing image (a) and image (b).

39. A method according to claim 35 to claim 38 wherein the sample solution contains cells or histological sections.
